# EUROPEAN PATENT SPECIFICATION

(45) Date of publication of patent specification: **30.11.94**

(51) Int. Cl.5: **C12P 21/08,** A61K 39/395, G01N 33/577, G01N 33/68, C07K 3/18

(21) Application number: **90115105.0**

(22) Date of filing: **06.08.90**

(54) **Antibodies to TNF binding protein I and F (ab) fragments thereof.**

(30) Priority: **06.08.89 IL 91229**
**06.04.90 IL 94039**

(43) Date of publication of application:
**13.02.91 Bulletin  91/07**

(45) Publication of the grant of the patent:
**30.11.94 Bulletin  94/48**

(84) Designated Contracting States:
**AT BE CH DE DK ES FR GB GR IT LI LU NL SE**

(56) References cited:
**EP-A- 0 308 378**
**EP-A- 0 334 165**

**PROCEEDINGS OF THE NATL. ACADADEMY OF SCIENCES USA, vol. 87, no. 8, April 1990, Washington, DC (US); M. BROCKHAUS et al., pp. 3127-3131&NUM;**

**JOURNAL BIOL. CHEM., vol. 265, no. 3, 25 January 1990, Baltimore, MD (US); H. ENGEL-MANN et al., pp. 1531-1536&NUM;**

(73) Proprietor: **YEDA RESEARCH AND DEVELOP-MENT COMPANY, LTD.**
**P.O. Box 26**
**Rehovot (IL)**

(72) Inventor: **Wallach, David**
**24 Borochov Street**
**Rehovot (IL)**
Inventor: **Engelmann, Harmut**
**Joseph Lutz-Weg 35**
**D-8000 München 70 (DE)**
Inventor: **Aderka, Dan**
**4 Avivim Street**
**Holon (IL)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

This invention relates to antibodies against Tumor Necrosis Factor (TNF) Binding Protein I (hereinafter TBP-I) and to F(ab) fragments thereof, and to the use of said antibodies and fragments in diagnostic assays or as agents for either inhibiting or mimicking the effects of TNF on cells.

Tumor Necrosis Factor (TNF-$\alpha$) and Lymphotoxin (TNF-$\beta$) (hereinafter, TNF refers to both TNF-$\alpha$ and TNF-$\beta$) are cytokines which have many effects on cells (Wallach, D. (1986) in: Interferon 7 (Ion Gresser, Ed.), pp. 83-122, Academic Press, London, and Beutler, B. and Cerami, A. (1987) New England J. Med. 316: 379-385). Both TNF-$\alpha$ and TNF-$\beta$ initiate their effects by binding to specific coil surface receptors. Some of the effects are likely to be beneficial to the organism: they may destroy, for example, tumor cells or virus infected cells and augment antibacterial activities of granulocytes. In this way, TNF contributes to the defense of the organism against infectious agents and to recovery from injury. But, quite clearly, both TNF-$\alpha$ and TNF-$\beta$ have also effects which can be extensively deleterious. There is evidence that over production of TNF-$\alpha$ can play a major pathogenic role in several diseases. Thus effects of TNF-$\alpha$, primarily on the vasculature, are now known to be a major cause for symptoms of septic shock (Tracey, K.J. et al. (1986) Science 234: 470-474). In some diseases, TNF may cause excessive loss of weight (cachexia) by suppressing activities of adipocytes and by causing anorexia and TNF-$\alpha$ was thus called cachectin.

There is therefore a necessity in finding out ways to eliminate or antagonize endogenously formed or exogenously administered TNF. One attempt in this direction was the isolation from human urine of the TNF Binding Protein called TBP-I and shown to be able to antagonize the effects of TNF. This antagonism was determined both by measuring reduction of the cytotoxic activity of TNF, as well as by measuring interference of TNF binding to its receptors.

The protein TBP-I was first described in our European Patent Application EP 308,378 published on March 22, 1989, in which was disclosed a process for its purification to homogeneity from human urine by chromatography on CM-Sepharose followed by high performance liquid chromatography (HPLC) on Mono Q and Mono S columns and reversed-phase HPLC. The homogeneous TBP-I thus obtained had an apparent molecular weight of about 27,000 in sodium dodecyl sulfate (SDS)-polyacrylamide gel electrophoresis (PAGE) under both reducing and nonreducing conditions. Homogeneity of the purified protein was confirmed by microsequence analysis which revealed a single N-terminal sequence: Asp-Ser-Val-Cys-Pro-.

TBP-I was shown to protect cells from TNF toxicity at concentrations of a few nanograms per ml and to interfere with the binding of both TNF-$\alpha$ and TNF-$\beta$ to cells, when applied simultaneously with these cytokines. Further examination of the mechanism by which TBP-I functions revealed that TBP-I does not interact with the target cell, but rather blocks the function of TNF by binding TNF specifically, thus competing for TNF with the TNF receptor.

Consequently to this finding we attempted an alternative approach for the purification of TBP-I, whereby urinary proteins or fractions thereof were applied on a column of immobilized TNF and, after removal of unbound proteins, the proteins which bound to the column were eluted, in bioactive form, by a decrease of the pH. In SDS PAGE analysis, most of the protein in the eluate migrated as a single broad hand with apparent molecular size of 30,000±2,000.

When applied to further fractionation by reversed-phase HPLC, the proteins eluting from the TNF column showed the presence of two active components: one, TBP-I, eluting as expected at 27% acetonitrile and, in addition, a second TNF-binding protein, called TBP-II, eluting at a somewhat higher acetonitrile concentration (31%). Both proteins provide protection against the in vitro cytocidal effect of TNF and both bind TNF-$\beta$ less effectively than TNF-$\alpha$. Although in SDS PAGE analysis the two proteins, TBP-I and TBP-II, appeared to have a very similar molecular size, they could clearly be distinguished from each other by lack of immunological cross reactivity, differing N-terminal amino acid sequences and differing amino acid compositions. TBP-I and TBP-II are structurally related to two molecular species of the cell surface TNF receptors, the type I and type II receptors, respectively.

The present invention provides polyclonal and monoclonal antibodies specific for the TNF-binding protein TBP-I which have the following properties:

a) they block the effect of TNF on specific cells. This blocking effect is obtained using the antibodies or F(ab) fragments thereof, taking advantage of the ability of said antibodies to block the binding of TNF to cells, apparently through interaction with structurally related cell surface TNF-binding proteins, i.e. TNF receptors; and

b) they mimic certain effects of TNF on specific cells. This mimicking effect of the antibodies is most likely due to activation of the receptors for TNF, upon their juxtaposition by the divalent antibodies, in a way which is similar to their activation by TNF itself.

The invention also comprises salts, functional derivatives and active fractions of the antibodies and of the F(ab) fragments thereof. As used herein, the term "salts" refers both to salts of carboxyl groups and to acid addition salts of amino groups of the protein molecule. "Functional derivatives" as used herein covers derivatives which may be prepared from the functional groups which occur as side chains on the residues or the N- or C-terminal groups, by means known in the art. As "active fractions" of the antibodies and their F(ab) fragments, the present invention covers any fragment or precursors of the polypeptide chain of said protein molecules alone or together with associated molecules or residues linked thereto, e.g. sugar or phosphate residues, or aggregates of the protein molecule or the sugar residues by themselves.

The invention further relates to the use of said antibodies and of F(ab) fragments thereof, and salts, functional derivatives or active fractions of said antibodies and fragments, as pharmaceutical agents both for mimicking and blocking effects of TNF on part or all cells in the human body.

Another aspect of the invention features the diagnostic use or tests for measuring either the TBP-I or the antisera against it, based on determining the interaction of the antibodies with the TBP-I. These diagnostic uses are of two kinds: (a) detecting endogenously produced antibodies to TBP-I in body fluids, to determine the extent to which such antibodies, by mimicking or blocking the effects of TNF, contribute to pathological manifestations of diseases, and (b) quantifying the levels of TBP-I in body fluids to detect or measure over- or under-production of this protein in any disorder characterized by abnormal production of said protein.

The figures show:

Figure 1 shows the Western blot analysis of the binding of rabbit antisera to TBP-I and TBP-II to the two species of TBP.

Figure 2 shows ELISA for the binding of rabbit antisera against TBP-I and TBP-II to the two species of TBP.

Figure 3 shows the inhibition of the binding of radiolabelled TNF to different cell lines with rabbit antisera to TBP-I and TBP-II.

Figure 4 shows the protection of cells from TNF cytotoxicity after preincubation with monovalent F(ab) fragments of antibodies to TBP-I.

Figure 5 shows the cytocidal effect of antibodies to TBP-I (A) and of TNF (B) on different cell lines.

Figure 6 shows the enhancement of $PGE_2$ synthesis by the antibodies to TBP-I and by TNF in different cell lines.

Figure 7 shows the growth stimulatory effect of the antibodies to TBP-I (A) and of TNF (B) on human fibroblasts and its reversion by IFN-gamma.

Figure 8 shows the antichlamydial effect of the antiserum to TBP-I, enhancement of the effect by IFN-gamma and its abolition at a high tryptophan concentration.

Figure 9 shows the lack of cytocidal activity in monovalent F(ab) fragments of the antibodies to TBP-I and recovery of that activity by cross-linking the F(ab) fragments with anti-immunoglobulin antibodies.

Figure 10 shows the morphology of SV80 cells after "pulse" treatment with monovalent F(ab) fragments of antibodies to TBP-I and further incubation in the presence or absence of anti-immunoglobulin antibodies.

Figure 11 shows the epitope mapping of TBP-I by cross-competition analysis with different monoclonal antibodies and correlation with the ability of the antibodies to suppress binding of TNF to HeLa cells and to mediate TNF-like cytotoxicity.

Figure 12 shows the levels of TBP-I in the sera of healthy individuals and of systemic lupus erythematosus (SLE) patients.

Figure 13 shows the levels of TBP-I in the sera of healthy individuals and of cancer patients.

The antibodies of the present invention provide a new approach for the modulation of the TNF activity, and may be used both to inhibit and to mimic effects of TNF on specific subsets of cells, depending on the molecular form of the antibodies, specifically on their valence: monovalent forms of the antibodies (e.g. F-(ab) fragments) being inhibitory and multivalent forms being able to mimic at least part of the effects of TNF. They are, thus, suitable as pharmaceutical agents both for mimicking and blocking TNF effects on cells.

The functional interaction of the antibodies of the present invention with TBP-I provides also a new diagnostic tool, based on immunoassays such as radioimmunoassay, ELISA etc., for the detection of over- or under-production of TBP-I by cells in the body in certain disorders. Thus, the level of TBP-I in sera of patients with different types of cancer or suffering from autoimmune disorders, such as systemic lupus erythematosus (SLE), can be determined this way. In an inverse approach, antibodies against TBP-I, when produced endogenously in the body, will be measured with the use of purified TBP-I. Detecting such autoantibodies, when formed in certain autoimmune disorders, is of extreme importance, since their ability to mimic or inhibit the effects of TNF surely has far-reaching bearing on the pathological syndromes of said disorders.

The antibodies may be either polyclonal or monoclonal. They may be raised in rabbits, mice or other animals or tissue cultured cells derived thereof or can be products of cells of human origin. They may also be produced by recombinant DNA technology either in a form identical to that of the native antibody or as chimeric molecules, constructed by recombination of antibody molecules of man and animal origins or in other forms chosen to make the antibodies most suitable for use in therapy.

For the preparation of the antibodies, either purified TBP-I or one or more synthetic peptides identical to the known sequence of a fragment thereof, e.g. to the N-terminal protein sequence, may be used to immunize animals. A further possibility is to fuse one of the possible nucleotide sequences coding for a fragment of TBP-I to the gene coding for Protein A, to express the fused Protein A-TBP-I gene in E. coli, to purify the fused protein by affinity chromatography on IgG Sepharose column and then to use it to immunize animals.

The monoclonal antibodies of the present invention are prepared using conventional hybridoma technique (Kohler et al. (1975) Nature 256:495; Kohler et al. (1976) Eur. J. Immunol. 6:511). After immunization, spleen cells alone or together with lymph node cells of the immunized animals are isolated and fused with a suitable myeloma cell line. After fusion, the resulting hybridoma cells are selectively maintained in HAT medium and then cloned. The hybridoma cells obtained through such a selection are then assayed to identify clones which secrete antibodies capable of binding TBP-I. After identification, the desired clones are grown in bulk, either in suspension culture or in ascitic fluid, by injecting the cells into the peritoneum of suitable host mice. The monoclonal antibodies produced by the hybridomas are then isolated and purified.

The monoclonal antibodies may also be immobilized and used for the purification of the TBP-I in affinity purification procedure using an immunoadsorbent column.

The antibodies of the invention are found to inhibit the binding of TNF to cells. Antisera to TBP-I block the binding of TNF to the cervical carcinoma HeLa cells and to the breast carcinoma MCF7 cells, but not to the histiocytic lymphoma U937 cells, while antisera against TBP-II have the inverse specificity. In the chronic myeloid leukemia K562 cells both antisera have inhibitory effects.

F(ab) fragments of the anti-TBP-I antibodies are found to block the effect of TNF on cells of a line to which TNF binding is blocked by the antiserum comprising said antibodies. However, the intact antibody molecules are found to exert the inverse effect; they by themselves elicit in cells a cytotoxic effect identical to the effect induced by TNF. This ability of antibodies apparently directed against cell surface receptors to mimic the effect of the agonist, as opposed to the lack of ability of the F(ab) fragments of the antibody to do so, is mostly a consequence of the ability of the divalent antibody molecule to cause clustering of the receptor molecules. This clustering somehow results in activation of the receptors in a way which is similar or identical to the activation of the receptors by TNF itself. Whatever the mechanisms involved, the fact that the antibodies can either mimic or inhibit the function of TNF, depending on the exact molecular form of the antibody applied, implies that these antibodies can practically serve as inhibitory or mimicking agents to TNF. Furthermore, since the antibodies to TBP-I and TBP-II bind to two different receptors for TNF, which are expressed on different cells exhibiting different responses to TNF and perhaps having also a different function even when expressed in the same cell, these inhibitory and mimicking effects of the antibodies to TBP-I and TBP-II can be applied for modulating the response to TNF, i.e. augmenting specifically beneficial effects and suppressing specifically deleterious effects of this cytokine.

As mentioned before, TBP-I and TBP-II are structurally related to the type I and type II cell surface receptors, respectively. The antibodies to TBP-I and TBP-II block specifically the binding of TNF to one of the two receptors and can be applied to immunoprecipitate the receptors. The antibodies to TBP-I also induce effects characteristic of TNF in cells which express the immunologically cross-reactive cell surface receptors.

The present invention further relates to pharmaceutical compositions comprising a pharmaceutically acceptable carrier and an antibody to TBP-I or an F(ab) fraction thereof or salts, functional derivatives, precursors or active fractions thereof or mixtures of any of the foregoing, as active ingredient. The way of administration can be via any of the accepted modes of administration for similar agents and will depend on the condition to be treated. The pharmaceutical compositions are prepared for administration by mixing the protein or its derivatives with physiologically acceptable carriers, stabilizers and excipients, and prepared in dosage form, e.g. by lyophilization in dosage vials. The amount of active compound to be administered will depend on the route of administration, the disease to be treated and the condition of the patient.

The invention will now be illustrated by the following non-limiting examples:

4

## EXAMPLE 1: Purification of TBP-I

### 1.1 Preparation of the urine concentrate

A pool of 200 l urine from healthy male donors or from healthy postmenopausal women was subjected to microfiltration on a Pellicon membrane with a pore size of 0.45 $\mu$m. The filtrate was concentrated by ultrafiltration using a Pellicon membrane with a molecular weight cut-off of 10 kDa to a final volume of 500 ml. The concentrate was dialyzed against phosphate buffered saline containing 1 mM benzamidine and 0.1% sodium azide.

### 1.2 Affinity purification of TBP-I on a column of immobilized TNF

Recombinant TNF-$\alpha$ was brought to a concentration of 7.2 mg/ml, then equilibrated with PBS containing 0.02% sodium azide and coupled to Affigel 10 (3.6 mg to 0.5 ml beads). A sample of 250 ml of the concentrate of urinary proteins of step 1.1 was applied to a column constructed from the beads of the immobilized TNF at a flow rate of 0.2 - 0.3 ml/min. at 4°C. Unbound proteins were removed by washing with PBS and the bound proteins were then eluted by applying a solution of 25 mM citric acid, 100 mM NaCl and 0.02% sodium azide, at pH 2.5. The specific bioactivity (inhibition of TNF toxicity) of the eluted proteins was about 20,000 fold higher than that of the crude urinary proteins. In SDS PAGE analysis most of the protein in the eluate migrated as a single broad band with apparent molecular size of about 30,000±2,000.

### 1.3 Reversed-phase high pressure liquid chromatography (HPLC)

Further fractionation of the affinity purified proteins of step 1.2 was by reversed-phase HPLC on an Aquapore RP300 column (4.6x30 mm, Brownlee Labs), first preequilibrated and then washed with 0.3% aqueous trifluoroacetic acid (TFA) (Buffer F) until a stable baseline was obtained by the fluorescamine detection system. Pooled active fractions eluted from the affinity TNF column of step 1.2 were applied on the column, elution was performed at a flow rate of 0.5 ml/minute with linear gradients of acetonitrile in Buffer F (0-20% for 5 minutes, followed by 20-50% for 60 minutes and finally 50-80% for 5 minutes), and then the column was washed for 15 minutes with 80% acetonitrile. TBP-I eluted between 26% and 29% acetonitrile and TBP-II between 29% and 32% acetonitrile concentration.

## EXAMPLE 2: Preparation of polyclonal antibodies against TBP-I

For the immunization of rabbits, the animals were first injected subcutaneously with 5$\mu$g of TBP-I as emulsion in complete Freund adjuvant. Three weeks later they were injected again, intramuscularly, as emulsion in incomplete Freund adjuvant and then twice again subcutaneously as solution in PBS, at one week intervals. The rabbits were bled 10 days after the last immunization.

For the purification of immunoglobulins from the rabbit serum, saturated ammonium sulfate was added to 10ml serum to a final concentration of 50% saturation. After overnight incubation at 4°C, the immunoglobulins were precipitated by centrifugation. The pellet was washed twice with 50% ammonium sulfate, then solubilized in 10mM sodium borate 0.02% sodium azide at pH 9. The solution was then dialyzed extensively against the borate-azide solution. It was then applied for chromatography on HPLC Mono-Q column, from which the proteins were eluted with a gradient of 0-500 mM NaCl in the above borate-azide solution. The immunoglobulins eluted at a salt concentration of approximately 70 mM NaCl.

The antiserum to TBP-I suppressed the binding of $^{125}$I-TNF to HeLa cells by about 50% at a dilution of 1:6400. Antiserum to TBP-II was raised in rabbits in the same conditions and was shown to suppress the binding of $^{125}$I-TNF to U937 cells at a similar dilution. The extent of the immunological cross-reactivity of both antisera were examined by Western blot analysis. It showed that TBP-I and TBP-II are immunologicaly distinct: each antiserum recognized significantly only that species of TBP against which it had been raised.

For the Western blot analysis, the tested proteins were applied to SDS PAGE on 10% acrylamide gels and then blotted electrophoretically to a nitrocellulose sheet. The nitrocellulose sheet was incubated with 10% milk (v/v) in PBS, then briefly rinsed in PBS and further incubated with the tested antibodies in a multi-lane device. After incubation with either $^{125}$I-labelled Protein A or $^{125}$I-goat antimouse F(ab) fragments of IgG, both at $5.10^2$ CPM/ml, the unbound material was washed and the nitrocellulose sheet was exposed to autoradiography.

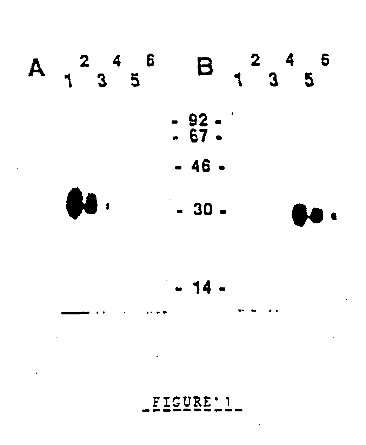

Figure 1 shows the Western blot analysis of the binding of antisera against TBP-I and TBP-II to the two proteins. TBP-I (A: lanes 1-6) and TBP-II (B: lanes 1-6) were applied to SDS PAGE at 2 $\mu$g/lane together with 2 $\mu$g BSA. Following electrophoresis the proteins were blotted electrophoretically to a nitrocellulose sheet which was then incubated with antiserum to TBP-I (lanes 1-3) or to TBP-II (lanes 4-6) at the following dilutions: lanes 1,4 - 1:100; lanes 2,5 - 1:500; lanes 3,6 - 1:2500. After incubation with the antibodies, the nitrocellulose sheet was incubated with $^{125}$I-labelled protein A and then washed and exposed to autoradiography.

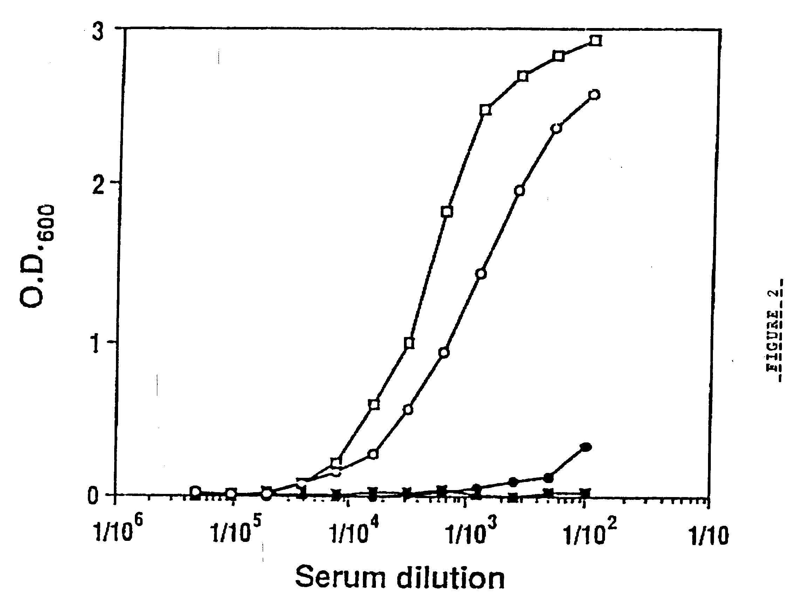

Similarly, when examining the interaction of the antisera and the proteins in ELISA, the antiserum against TBP-I was found to react with TBP-I at a dilution of up to 1:25,000, but did not react with TBP-II, not even at a dilution of 1:100. Figure 2 shows the results of ELISA for the binding of antisera against TBP-I and TBP-II to the two species of TBP. The binding of (□) antiserum against TBP-I to TBP-I, (■) antiserum against TBP-I to TBP-II, (●) antiserum against TBP-II to TBP-I, and (o) antiserum against TBP-II to TBP-II, is presented in terms of the absorbance of the color product in the horseradish peroxidase assay. The readings in a control test at which the antibodies were applied on wells coated with BSA were substracted. (The slight binding of the antiserum against TBP-II to TBP-I, observed in Fig. 2, could be shown to be due to contamination of the antiserum with antibodies to TBP-I, at low amounts, due to the presence of some TBP-I in the preparation of TBP-II used for immunization).

### EXAMPLE 3: Effects of the polyclonal antibodies on binding of TNF to cells

The antisera to TBP-I and TBP-II were diluted in Dulbecco's balanced salt solution (PBS$^+$) containing 0.5% BSA and 0.1% sodium azide (PBS/BSA) and then either directly or, in competition experiments, after incubation with a sample of TBP, applied for 2 h on the tested cells of the HeLa, MCF7, K562 and U937 cell lines. The cells were then rinsed and tested for binding of TNF.

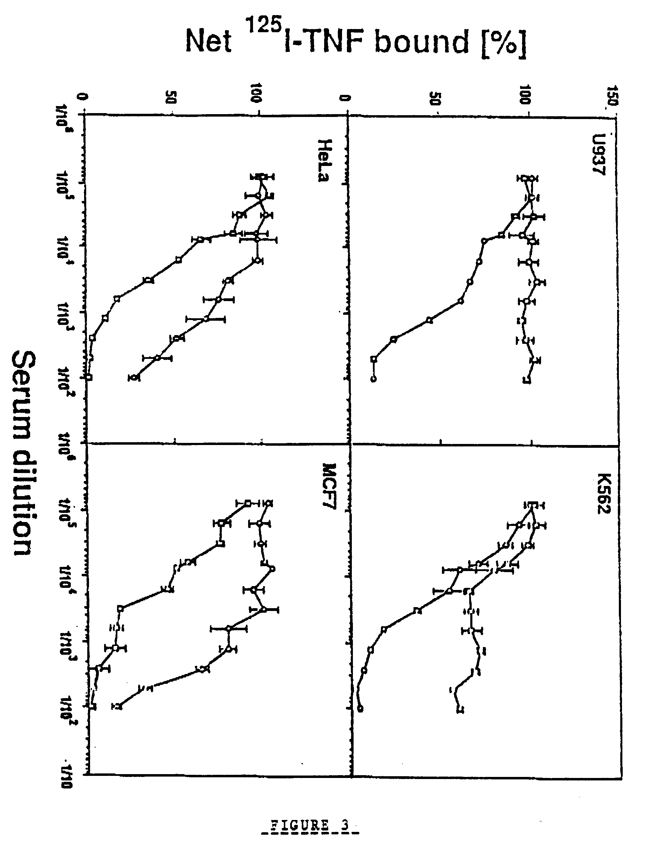

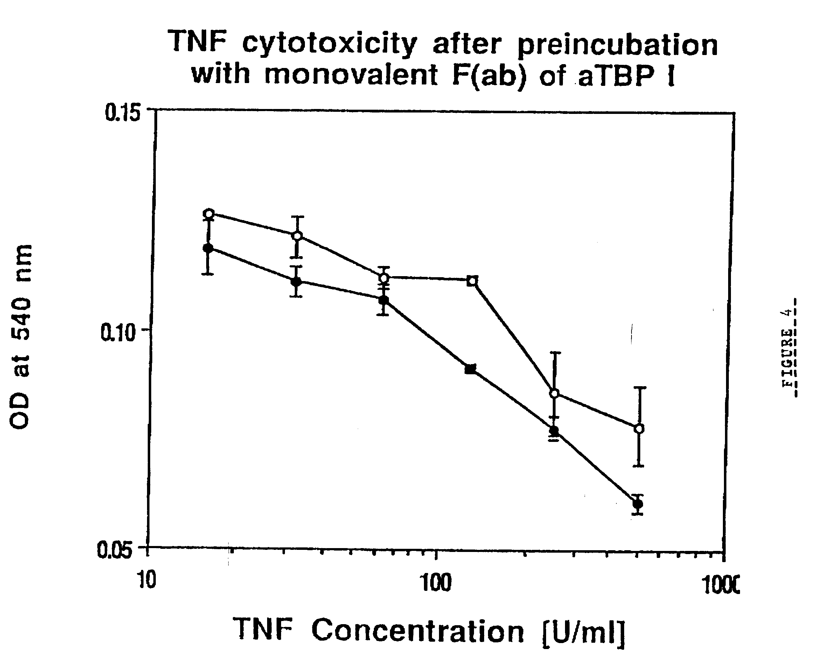

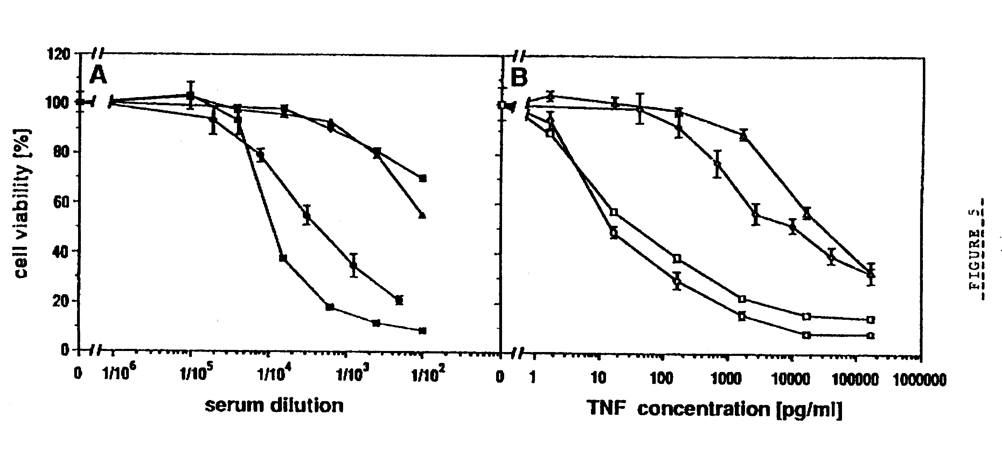

Figure 3 shows the inhibition of the binding of radiolabelled TNF to U937, K562, HeLa and MCF7 cells with antisera to TBP-I (o) and TBP-II (□). The net binding observed in the absence of antisera (100%) was in U937 cells - 2500 CPM, in K562 cells - 1500 CPM, in HeLa cells - 2400 CPM and in MCF7 cells - 1100 CPM. The results demonstrate that antisera against TBP-I and TBP-II interfere with the binding of TNF to cells; each affecting to different extent cells of different lines. The antiserum against TBP-I inhibits effectively the binding of TNF to HeLa and MCF7 cells, but has no effect on the binding of TNF to U937 cells and only little effect on the binding of TNF to K652 cells. Inversely, the antiserum against TBP-II blocks effectively the binding of TNF to the K562 and U937 cells, but inhibits the binding of TNF to the HeLa and MCF7 cells only at high concentrations. The effect of the antiserum against TBP II on the latter cells could be shown, by competition experiments, at which pure TBP-I and TBP-II were added to the serum, to be due to the presence of contaminating antibodies to TBP-I in this preparation of antiserum to TBP-II.

### EXAMPLE 4: F(ab) fragments of polyclonal antibodies to TBP-I

Immunoglobulins purified from the antiserum to TBP-I were exposed to digestion with papain, in the presence of cysteine/EDTA at an enzyme/substrate ratio of 1:100. The antibody digest was dialyzed against 10mM sodium acetate buffer, pH 5.5 and subjected to cation exchange HPLC on a Mono-S column. The bound proteins were eluted from the Mono-S column with a 0-300mM gradient of NaCl in 10mM sodium acetate buffer, which resulted in purification of the F(ab) fragments to homogeneity. Purity was verified by SDS PAGE analysis under reducing and non-reducing conditions. To test the effect of these fragments on the cytocidal activity of TNF, they were applied on HeLa cells, the cells were then rinsed to remove all unbound antibodies and exposed for 10 h to TNF, together with cycloheximide (25 $\mu$g/ml). Viability of cells was than determined by the neutral red uptake method.

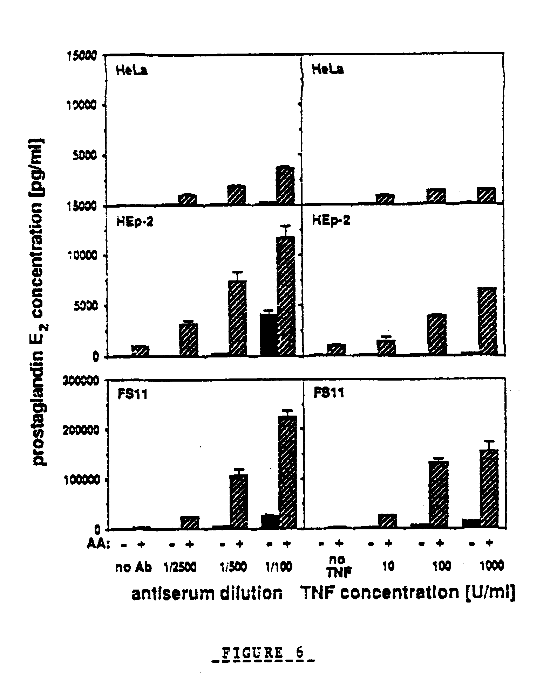

In Fig. 4: Protection from TNF cytotoxicity by the monovalent fragments of the antibodies to TBP-I: HeLa cells which were pretreated with the F(ab) fragments (3$\mu$g/ml (●) and, for comparison, cells treated in the same way with medium alone, (o) were further incubated for 10 h with TNF, at various concentrations, together with CHI (25 $\mu$g/ml). The results show that the F(ab) fragments of the antibodies to TBP-I protect HeLa cells from the cytocidal effect of TNF - a reflection of the fact that they interfere with the binding of TNF to the TNF receptors expressed on the surface of these cells.

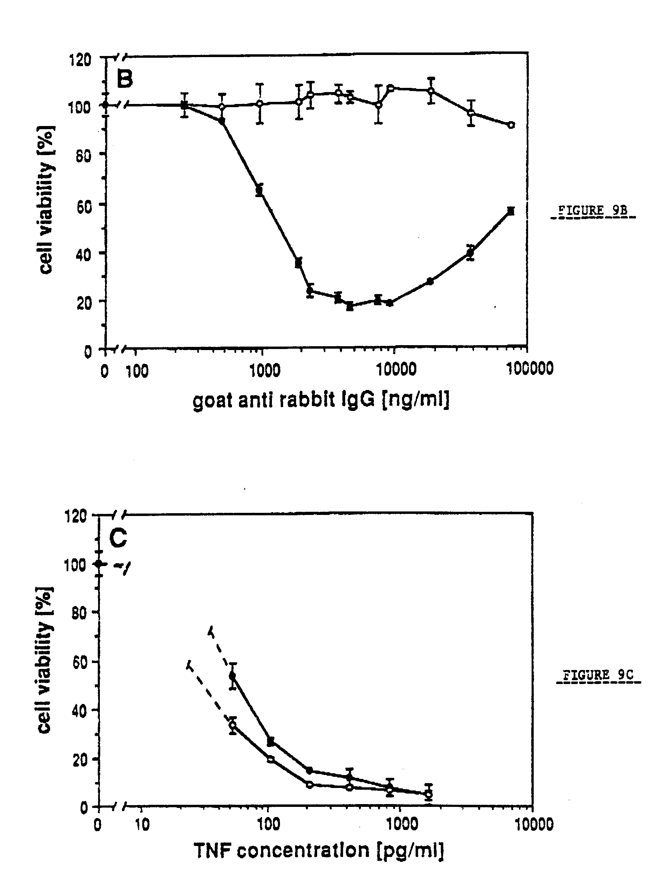

Similar results were obtained when the F(ab) fragments were applied on SV80 cells as illustrated in Fig. 9C, described in detail under Example 5, section f, below.

Table I shows that in the intact state antibodies to TBP-I have the inverse effect to that mediated by their F(ab) fragments; namely, they mimic the effect of TNF and are by themselves cytotoxic.

Table I

| TNF-like cytocidal effect of antibodies to TBP-I | |
|---|---|
| TNF (u/ml) | Cell Viability (%) |
| -- | 100 |
| 1 | 31 |
| 10 | 15 |
| 100 | 6 |
| 1000 | 3 |
| anti TBP serum (dilution) | |
| 1:6400 | 69 |
| 1:1600 | 37 |
| 1: 400 | 17 |
| 1: 100 | 9 |

TNF and the antiserum to TBP-I were applied on SV80 cells for 12 h together with cycloheximide. Viability of the cells was then determined by the neutral red uptake method (see also Fig. 5).

**EXAMPLE 5: Determination of bioactivities of the antibodies to TBP-I**

a. Cytotoxic activity

Cells were seeded 24 hr prior to assay in 96 well (microtiter plates) ($3 \times 10^4$ cells/well). The antibodies to TBP-I or TNF were applied in serial dilutions either in the presence or the absence of cyclohexi- mide (CHI) (25 $\mu$g/ml in the case of the HeLa cells and 50 $\mu$g/ml for the other cells). After an incubation period of 12 hr for the HeLa cells and 16 hr for all the others, cell viability was determined by the neutral-red uptake method. Cell viability is presented as the per cent ratio of the viability of cultures incubated with CHI alone (for cells tasted in its presence) or without additives.

Figure 5 shows the cytocidal effect of the antibodies to TBP-I (A) and of TNF (B) on SV80 (o,●) HeLa (□,■), FS11 (△,▲) and HEp-2 (◊,♦) cells. The antibodies and TNF were applied for 15 hr (12 hr for HeLa cells) together with CHI (25 $\mu$g/ml for HeLa and 50 $\mu$g/ml for all other cells). Cell viability was quantitated by measuring the uptake of neutral red dye. Viability or cells incubated with anti TBP-I at 1:200 in the absence of CHI was 99% in the SV80 cells, 97% in HeLa cells, 98% in FS11 cells and 96% in the HEp-2 cells. Normal rabbit serum in the range of concentrations of anti-TBP-I applied in this study had no effect in this experiment or in any of the other experiments presented below. All tests were performed in duplicates.

b. Induction of PGE$_2$ synthesis

Cells were seeded in 96-well microtiter plates ($5 \times 10^4$ cells/well). Ten hours thereafter TNF and the antibodies were applied in serial dilutions. After further incubation for 15 hr at 37°C, the cell growth medium was collected and replaced with fresh medium containing arachidonic acid ($5 \times 10^{-5}$ M). One hour later, the medium was collected again. The PGE$_2$ content of all samples was determined by an immuno-assay as described by Kaever et al., Prostaglandins, 35: 885-902, 1988. Figure 6 shows the enhancement of PGE$_2$ synthesis by the antibodies to TBP-I and by TNF in HeLa, HEp-2 and FS11 cells and its augmentation by arachidonic acid (AA, 5 $\mu$M).

c. Stimulation of fibroblast growth

Growth stimulation in fibroblasts due to TNF and the antibodies to TBP-I was determined essentially as described by Vilcek et al., J. Exp. Med., 163: 632-643, 1986. Human foreskin fibroblasts (strain FS11, passage 10-12) were seeded in 96-well microtiter plates ($10^4$ cells/well). Either TNF, or the antibodies to TBP-I, were applied 18 h later. The rate of thymidine incorporation into the cells after 3 days of further incubation was determined by applying $^3$H-thymidine to the cells (1 $\mu$Ci/well) and incubating them for another 16 h. The cells were then rinsed once with cold PBS, detached with trypsin, and the amount of label incorporated was determined by harvesting the cells onto glass fiber filters followed by liquid scintillation counting.

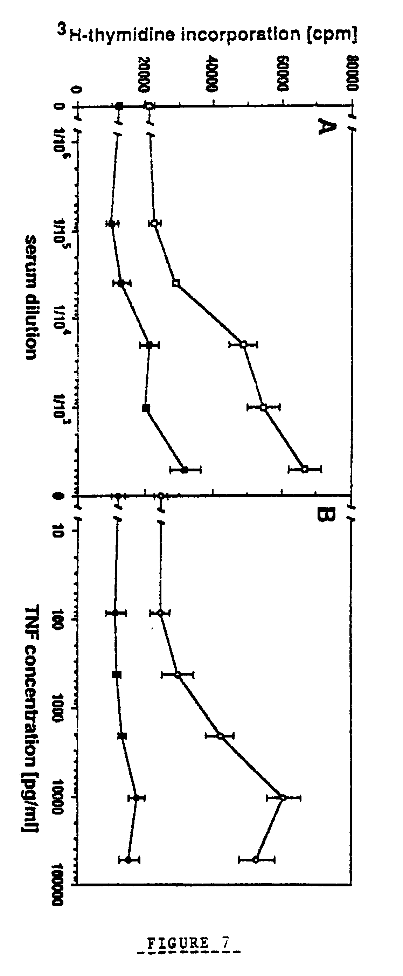

Figure 7 shows the growth stimulatory effect of the antibodies to TBP-I (A) and of TNF (B) on human fibroblasts and its reversion by IFN-gamma. Human foreskin fibroblasts (strain FS11) were incubated for 3 days with the antibodies to TBP-I. (□) or with TNF (o), in the presence (■,●) or absence (□,o) of IFN-gamma (250 U/ml). At the end of this incubation period the rate of $^3$H-thymidine incorporation was determined.

d. Inhibition of chlamydial growth

The effect of the antibodies to TBP-I on growth of Chlamydia trachomatis (L$_2$434/Bu) in the HEp-2 cells was determined, as described for the antichlamydial effect of TNF by Shemer-Avni et al., Infect. Immun. 56:2503-2506, 1988. The antibodies were applied on the HEp-2 cells at the indicated concentration either alone or together with IFN-gamma; first, 1 day before infection with the chlamydiae and again, at the same concentrations, immediately after infection. The yield of chlamydiae 2 days after infection was determined using an immunoperoxidase assay for the chlamydial antigens and expressed as inclusion forming units/ml (IFU/ml).

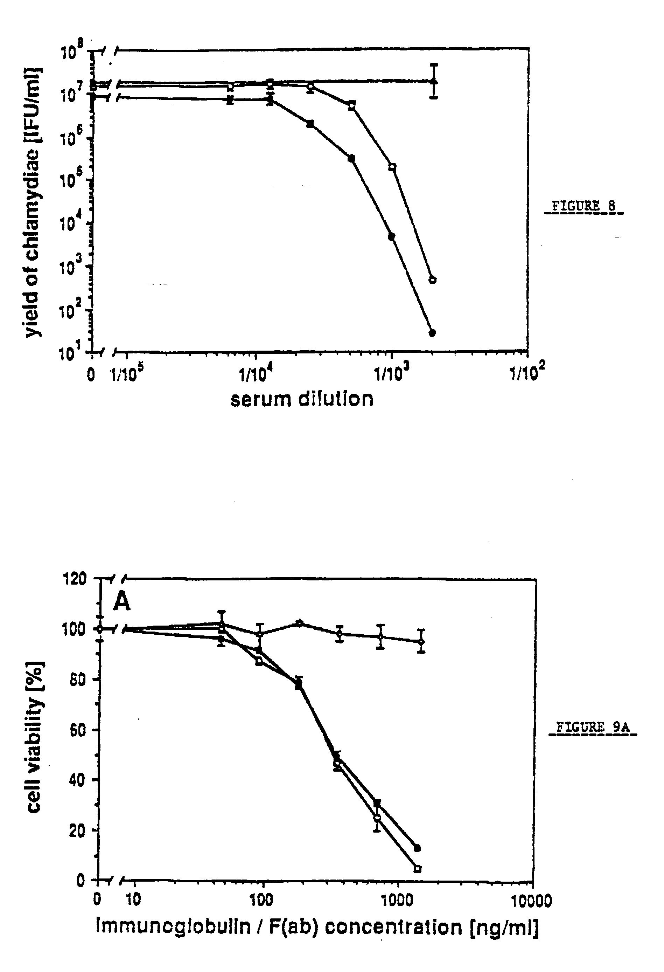

Figure 8 shows the antichlamydial effect of the antiserum to TBP-I, enhancement of the effect by IFN-gamma and its abolition at high tryptophan concentration. The effect of the antiserum, at the indicated dilutions, in the presence (●) or absence (o) of IFN-gamma (2 U/ml) was quantitated. Increase of tryptophan concentration, at the time of infection of the cells treated with anti-TBP-I from 10 μg/ml to 200 μg/ml abolished the antichlamydial effect (▲).

e. Antibodies to TBP-I have TNF-like effects

Applying the polyclonal antibodies to TBP-I on cells in the presence of the protein synthesis inhibitor cycloheximide (CHI) resulted, within a few hours, in extensive cytolysis. The cytocidal effect was complement-independent (data not shown) and appeared morphologically very similar to the cytocidal effect of TNF. It resembled the effect of TNF in several other respects as well:

(i) The sensitivity of different cell lines to polyclonal anti-TBP-I antibodies followed a similar pattern as their sensitivity to TNF. Thus, human foreskin fibroblasts (strain FS11) and HEp-2 cells, which are relatively resistant to TNF toxicity, were also much less sensitive to the toxicity of the antibodies (Fig. 5).

(ii) Like TNF, the antibodies failed to kill HeLa and SV80 cells in the absence of protein synthesis inhibitors (see cytotoxic activity under Section a above).

(iii) The sensitizing effect of protein synthesis inhibitors to the antibody-mediated cell killing was largely dependent on the timing of their application. Maximal cytotoxity could be observed when the inhibitors and the antibodies were applied simultaneously. Application of the inhibitors a few hours after the antibodies resulted in significantly reduced cell death (Table II). The same time-dependence was observed for the sensitization by such inhibitors to the cytocidal effect of TNF (Table II). Thus the antibodies, like TNF, could be either cytotoxic to cells or induce in them resistance to their own toxicity, depending on whether they were applied in the presence or absence of protein synthesis inhibitors.

(iv) Resistance to both the toxicity of the antibodies and of TNF was induced in the SV80 cells also by pretreatment with IL-1 (Table III). Furthermore, the antibodies and TNF could induce in these cells cross resistance to each other's toxicity (Table III).

In the following tables, a̅TBP-I stands for antibodies to TBP-I.

Table II

| Time-dependent sensitization of SV80 cells to the cytocidal effect of TNF or of antibodies to TBP-I, by CHI. | | |
|---|---|---|
| Time of CHI application | TNF(100u/ml) | a̅ TBP-I (1:200) |
| | Cell viability (%) | |
| Simultaneously with a̅ TBP-I/TNF | <1 | <1 |
| + 1 h | <1 | <1 |
| + 3 h | 41 | 48 |
| + 6 h | 77 | 73 |
| Not added | 100 | 100 |
| SV80 cells were incubated for 16 h with TNF or with the antibodies to TBP-I (a̅). CHI (50 $\mu$g/ml) was added to the culture at time zero or at 1, 3, or 6 h after application of TNF or of the antibodies (a̅). Cell viability was determined at the end of the incubation period by the neutral red uptake method. | | |

Table III

| Induction of resistance to the cytocidal effects of $\bar{a}$ TBP-I, or of TNF by TNF itself, $\bar{a}$ TBP-I and IL-1 | | | | |
|---|---|---|---|---|
| Treatment | Pretreatment (for 6 h) | | | |
| | - | TNF (100 U/ml) | IL-1 (10 U/ml) | $\bar{a}$ TBP-I (1:200) |
| | Cell-viability (%) | | | |
| CHI (50 $\mu$g/ml) | 100 | 91 | 95 | 85 |
| TNF ($10^4$ U/ml) + CHI | 8 | 82 | 82 | 71 |
| $\bar{a}$ TBP-I (1:200) + CHI | 9 | 89 | 84 | N.D. |

N.D. Not determined SV80 cells were treated for 1 h with TNF, IL-1, $\bar{a}$ TBP-I or without additives and incubated further for 6 h in medium alone to allow full recovery of the TNF receptors (pretreatment). The cells were then incubated for 12 h with TNF or with $\bar{a}$ TBP-I in the presence of CHI, or with CHI alone (treatment). While without any pretreatment, most cells were killed when incubated with TNF or with $\bar{a}$ TBP-I together with CHI, cells which were first incubated with TNF, IL-1 or $\bar{a}$ TBP-I in the absence of CHI were largely resistant to subsequent treatment with TNF or $\bar{a}$ TBP-I in the presence of CHI.

Further examination of the effect of antibodies to TBP-I, when applied on cells in the absence of protein synthesis blockers, revealed that under these conditions the antibodies mediated several non-cytocidal TNF-like effects. In the foreskin fibreblasts and HEp-2 cells, which are quite resistant to TNF-cytotoxicity, as well as in the TNF sensitive HeLa cells, the antibodies, similarly to TNF had a marked stimulatory effect on the synthesis of $PGE_2$ (Fig. 6). The effect of both was particularly prominent when arachidonic acid was added to the cells, suggesting that in both cases it reflects an increase, not in the release of arachidonic acid, but in its conversion to prostaglandin. An additional TNF-like effect of the antibodies in the foreskin fibroblasts was an enhancement of the incorporation of thymidine (Fig. 7), apparently reflecting stimulation of cell growth. Like the growth stimulatory effect of TNF, the stimulation of fibroblast growth by the antibodies was obliterated when the cells were treated simultaneously with IFN-gamma (Fig. 7).

In HEp-2 cells, TNF suppresses the growth of chlamydiae, obligate parasitic bacteria which grow intracellularly within membrane bound structures. As shown in Fig. 8, growth of chlamydiae in those cells was also markedly inhibited by the antibodies to TBP-I. Inhibition of chlamydial growth by TNF is synergistic with the antichlamydial effect of IFN-gamma and is largely abrogated when the HEp-2 cells are grown in the presence of increased concentrations of tryptophan. The antichlamydial effect of the antibodies to TBP-I was affected by IFN-gamma and tryptophan in a similar manner (Fig. 8).

f. The TNF-like activity of the antibodies to TBP-I correlates with their ability to crosslink the TNF receptor molecules

To explore the mechanisms for the TNF-like activity of antibodies to TBP-I, we tested the effect of monovalent F (ab) fragments of anti-TBP-I on cell function. Like the intact antibodies, the monovalent fragments effectively blocked the binding of radiolabelled TNF to cells, suggesting that they maintained the ability to bind to the cell surface TNF receptors ($EC_{50}$ was about 0.8 $\mu$g/ml for the intact antibodies and 1 $\mu$g/ml for the monovalent fragments). However, while in their intact form the antibodies were cytotoxic to CHI-treated SV80 cells at concentrations as low as 0.1 $\mu$g/ml, the monovalent F(ab) fragments of the antibodies did not exhibit any toxic effects (Fig. 9A). Indeed, by virtue of their ability to inhibit the binding of TNF to cells, the monovalent F(ab) fragments not only failed to kill the SV80 cells, but even had some inhibitory effect on their killing by TNF (Fig. 9C).

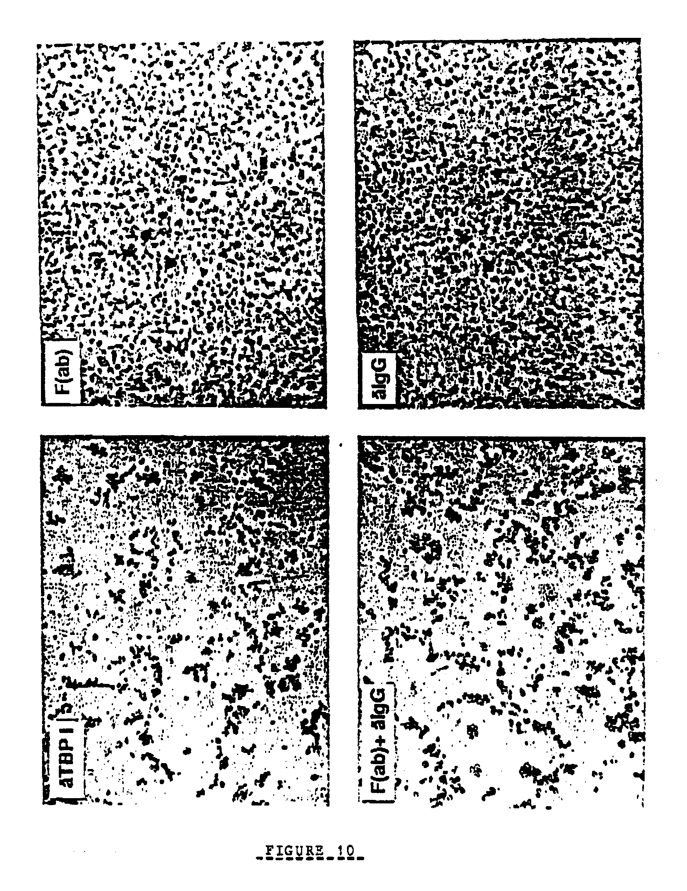

To check whether this loss of TNF-like activity in the fragmented antibodies was related to their monovalence we performed experiments to determine whether cross-linking of the F(ab) fragments would result in resurgence of their cytotoxic activity. It has been shown before that pulse-treatment of SV80 cells with TNF at 4°C, followed by incubation with CHI at 37°C, is sufficient to cause effective cell death. The intact antibodies to TBP-I were also cytotoxic under these conditions (compare solid and empty circles in Fig, 9A), while monovalent F(ab) fragments were not cytotoxic. However, then the F(ab)-pretreated cells were treated subsequently with goat antibodies to rabbit Ig to elicit cross-linking of the cell-bound antibody fragments, extensive cell death occurred (Figs. 9B, 10).

Figure 9A shows the lack of cytocidal activity of monovalent F(ab) fragments of the antibodies to TBP-I (◊), and the cytocidal effect of the anti-TBP-I immunoglobulins (o) at different concentrations, when applied to SV80 cells for 16 h together with CHI (50 $\mu$g/ml), and of "pulse" treatment with the anti-TBP-I immunoglobulins (●). Titration of the cytocidal effect of "pulse" treatment with the anti-TBP-I immunoglobulins was performed as follows: The cells were incubated with the antibodies, at the indicated concentrations, for 2 h at 4°C, rinsed and incubated further at 37°C with CHI (50 $\mu$g/ml) with no further addition of the antibodies.

Figure 9B shows the effect of goat antibodies to rabbit immunoglobulins when applied to SV80 cells which had been "pulse" treated with monovalent F(ab) fragments of the anti-TBP-I (●) or to untreated cells (o) (5 $\mu$g/ml). The "pulse" treatment with the F(ab) fragments was performed as described above for the intact immunoglobulins in Figure 9A.

Figure 9C shows the protection from TNF cytotoxicity by the monovalent fragments of the antibodies to TBP-I: SV80 cells which were "pulse" treated with the F(ab) fragments, as in Figure 9B and, for comparison, cells treated in the same way with medium alone were further incubated for 16 h with TNF, at various concentrations, together with CHI (50 $\mu$g/ml).

Figure 10 shows the morphology of SV80 cells after "pulse" treatment with monovalent F(ab) fragments of anti-TBP-I and further incubation in the presence or absence of anti immunoglobulin antibodies. Anti-TBP-I immunoglobulins and their monovalent F(ab) fragments were applied to the SV80 cells for 2 hr in the cold at a concentration of 3 $\mu$g/ml followed by rinsing and incubation for 16 hr at 37°C in the presence of CHI with or without goat anti-rabbit IgE (4.5 $\mu$g/ml). All other conditions of the assay were as for Fig. 7.

Photographs were taken at a magnification of x125 after staining the cells with neutral red.

## EXAMPLE 6: Monoclonal antibodies to TBP-I

Production of the monoclonal antibodies

Female Balb/C mice (8 weeks old) were injected with 1 μg purified TBP-I in an emulsion of complete Freund's adjuvant into the hind foot pads, and three weeks later, subcutaneously into the back in incomplete Freund's adjuvant. The other injections were given in weekly intervals, subcutaneously in PBS. Final boosts were given 4 days (i.p.) and 3 days (i.v.) before the fusion with 9.0 μg of TBP-I in PBS. Fusion was performed using NSO/Mr cells and lymphocytes prepared from both the spleen and the local lymphocytes of the hind legs as fusion partners. The hybridomas were selected in DMEM supplemented with HAT, 15% horse serum and gentamycin 2 μg/ml. Hybridomas that were found to produce antibodies to TBP-I were subcloned by the limiting dilution method and injected into Balb/C mice that had been primed with pristane for the production of ascites. Immunoglobulins were isolated from the ascites by ammonium sulfate precipitation (50% saturation) and then dialyzed against PBS containing 0.02% azide. Purity was approximately 60% as estimated by analysis on SDS-PAGE and staining with commassie blue. The isotypes of the antibodies were defined with the use of a commercially available ELISA kit (Amersham, U.K.).

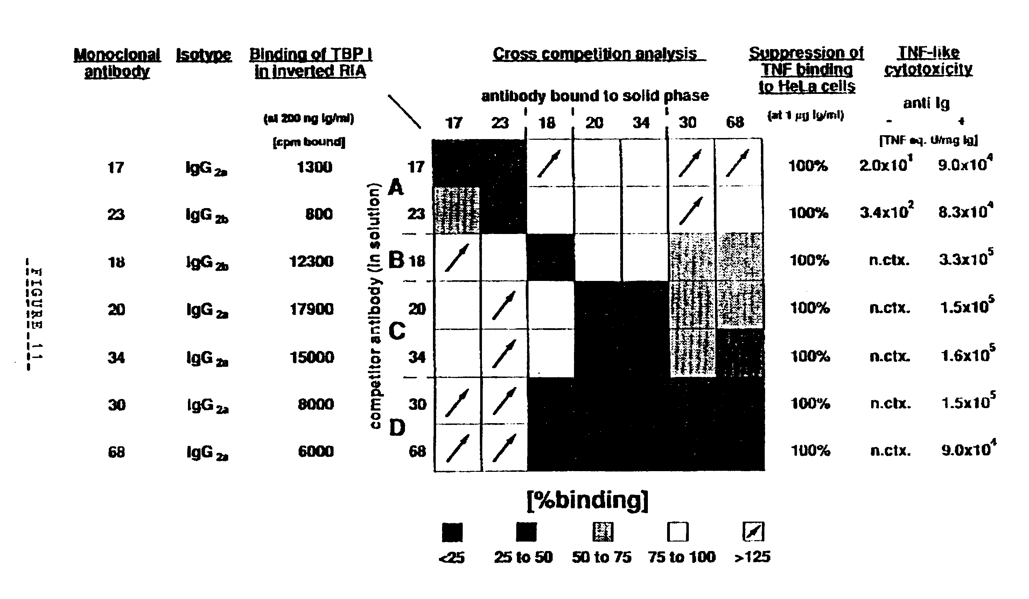

Several positive clones were obtained for further studies and characterized. Some of the isolated subclones with their isotype and binding of TBP-I in inverted RIA are listed in Table IV. The isotypes end other characteristics of the monoclonal antibodies are given in Fig. 11.

Hybridomas TBP-I 18-1 and TBP-I 34-6 were deposited with the Collection Nationale de Cultures de Microorganismes, Institut Pasteur (CNCM), 25, rue du Docteur Roux, 75724 Paris CEDEX 15, France on March 12, 1990 and were assigned No. I-926 and No. I-927, respectively.

## Table IV

Subclones producing monoclonal antibodies to TBP-I

| Clone number | Screening with iRIA [CPM] | Screening of subclone with iRIA [CPM] | Isotype |
|---|---|---|---|
| 7.5 | 10000 | 2900 | IgG2a |
| .8 | | 2800 | IgG2a |
| .9 | | 5200 | IgG2a |
| 8.1 | 13440 | 10800 | IgG1 |
| .3 | | 11300 | IgG1 |
| .11 | | 11000 | IgG1 |
| 13.2 | 28300 | 13600 | IgG2a |
| .3 | | 15000 | IgG2a |
| .5 | | 12900 | IgG2a |
| 14.6 | 17000 | 5400 | IgG2b |
| .7 | | 6200 | IgG2b |
| .13 | | 5700 | IgG2b |
| 16.1 | 29000 | 18000 | IgG2a |
| .3 | | 16000 | IgG2a |
| .7 | | 17500 | IgG2a |
| 17.2 | 7076 | 2100 | IgG2a |
| .7 | | 2100 | IgG2a |
| .9 | | 2200 | IgG2a |
| 18.1 | 28000 | 28800 | IgG2b |
| .2 | | 27200 | IgG2b |
| .3 | | 29800 | IgG2b |
| 20.2 | 46000 | 29800 | IgG2a |
| .5 | | 32600 | IgG2a |
| .11 | | 31400 | IgG2a |
| 23.1 | 5300 | 1700 | IgG2a |
| .3 | | 1500 | IgG2a |
| .5 | | 1900 | IgG2a |
| 29.1 | 4900 | 1600 | IgG2a |
| .4 | | 1200 | IgG2a |
| .5 | | 1100 | IgG2a |
| 30.1 | 48000 | 26700 | IgG2a |
| .3 | | 27500 | IgG2a |
| .5 | | 26700 | IgG2a |

Table IV cont.

### Subclones producing monoclonal antibodies to TBP-I

| Clone number | Screening with iRIA [CPM] | Screening of subclone with iRIA [CPM] | Isotype |
|---|---|---|---|
| 34.5 | 29000 | 32800 | IgG2a |
| .7 | | 34200 | IgG2a |
| .12 | | 32300 | IgG2a |
| 39.1 | 4400 | 31800 | IgG2a |
| .6 | | 31700 | IgG2a |
| .8 | | 31500 | IgG2a |
| 68.1 | 46700 | 28900 | IgG2a |
| .4 | | 28000 | IgG2a |
| .6 | | 27500 | IgG2a |
| 79.6 | 2100 | 5000 | IgG2a |
| .7 | | 1900 | IgG2a |
| .8 | | 1900 | IgG2a |
| 80.1 | 2200 | 1900 | IgG2b |
| .4 | | 2100 | IgG2b |
| .13 | | 1800 | IgG2b |
| 83.2 | 1400 | 1600 | IgG2a |
| .7 | | 1700 | IgG2a |
| .9 | | 1600 | IgG2a |
| 86.1 | 2300 | 1500 | IgG2a |
| .7 | | 1400 | IgG2a |
| .8 | | 1600 | IgG2a |
| 91.2 | 1700 | 1900 | IgG2a |
| .3 | | 1500 | IgG2a |
| .6 | | 1600 | IgG2a |
| 92.1 | 2600 | 1900 | IgG2a |
| .2 | | 1600 | IgG2a |
| .6 | | 1500 | IgG2a |

**EXAMPLE 7: Inverted Radioimmunoassay (iRIA) for the detection of the monoclonal antibodies to TBP-I**

This assay was used for estimating the level of the anti-TBP antibodies in the sera of the immunized mice and for screening for the production of the antibodies by hybridomas. PVC, 96-well microtiter plates (Dynatech 1-220-25) were coated for 12 hr at 4°C with affinity purified goat anti mouse F(ab) im-

munoglobulins (Biomakor, Israel 10 $\mu$g/ml in PBS containing 0.02% NaN$_3$), then blocked for 2 hr at 37°C with 0.5% BSA in PBS supplemented with 0.05% Tween 20 (Sigma) and 0.02% NaN$_3$ (blocking buffer) and washed 3 times with PBS containing 0.05% Tween 20 and 0.02% NaN$_3$ (washing buffer). Serum samples, in serial dilutions, or samples of hybridoma growth media (50 $\mu$l) were applied into the wells for 2 hr at 37°C. The plates were rinsed with washing buffer and $^{125}$I-labelled TBP-I (10,000 cpm, in blocking buffer) was applied into the wells. After further incubation of 2 hr at 37°C, the plates were washed and the amount of label which bound to individual wells was determined in the gamma-counter.

### EXAMPLE 8: Epitope mapping of TBP-I by cross competition analysis with monoclonal antibodies

Polyvinylchloride (PVC), 96-well microtiter plates were coated, as described above, with purified monoclonal antibodies (mAbs) to TBP-I (25$\mu$g/ml) and, following rinsing and blocking, samples of $^{125}$I-labelled TBP-I (50,000 cpm per well) which had been pre-incubated for 2 hr, at 37°C, with the same or another monoclonal antibody to TBP-I (at 1 $\mu$g/ml) were put into the wells. The plates were incubated overnight at 4°C, washed and the radioactivity bound to each well was determined in the gamma-counter The results in Fig. 11 are expressed in percent of the control well where TBP-I was allowed to bind in the absence of competing mAbs.

Figure 11 shows the epitope mapping of TBP-I by cross competition analysis with 7 different mAbs to TBP-I and correlation with the ability of the antibodies to suppress binding of TNF to HeLa cells and to mediate TNF-like cytotoxicity. Binding of radiolabelled TBP-I to the mAbs (applied at saturating concentration, 200 ng/ml) in the inverted RIA, cross competition analysis of the mAbs for their binding to TBP-I and determination of the effect of the mAbs on the binding of TNF to HeLa cells were carried out as described. The TNF-like cytotoxicity of the antibodies was measured in two ways. In the first, (-anti Ig), SV80 cells were incubated 16 hr it 37°C with the mAbs in the presence of CHI (50 $\mu$g/ml). In the second, (+ anti Ig), the effect of crosslinking by anti-immunoglobulin antibodies on the cytotoxicity of the mAbs was tested. In this case, the SV80 cells were pulse-treated with the antibodies (2 hr at 4°C), rinsed, and further incubated (16 hr at 37°C) with rabbit anti-mouse F(ab)$_2$ antibodies (+ anti Ig) in the presence of CHI. Cell viability was determined by the neutral red uptake method. The cytotoxic activity of the antibodies is expressed in TNF equivalent units per mg immunoglobulin, where one TNF equivalent unit is defined as the amount of antibody exerting the same cytotoxicity as 1 U/ml TNF (16 pg/ml) (n.ctx - not cytotoxic).

The epitope mapping of TBP-I by cross competition analysis with the 7 mAbs suggests that these antibodies bind to four topologically distinct areas on the molecule (A - defined by mAbs 17 and 23, B - by 18, C - by 20 and 34, and D - by 30 and 68 (Fig. 11). An exceptional competition pattern was observed for the antibodies associated with epitope region D. The antibodies not only competed effectively with each other but also with the antibodies belonging to regions B and C. Moreover, the antibodies defining regions B and C competed, although less effectively, with the antibodies which bind to epitope D. One possible interpretation for this phenomenon is that epitopes B and C, while spatially distinct, overlap both with the epitope region represented by group D. An alternative possibility is that binding of an antibody to the epitope region D imposes a conformational change on TBP-I which prevents the binding of the antibodies recognizing determinants in B and C. The latter hypothesis seems consistent with the fact that the antibodies defining epitope D were the only ones found to recognize TBP-I after its denaturation with SDS in the presence of $\beta$-mercaptoethanol (not shown).

When applied to HeLa cells, the seven monoclonal antibodies to TBP-I described above, as well as ten other monoclonal antibodies to TBP-I all had a marked inhibitory effect on TNF binding (Fig. 11), implying that all the different epitopes which they recognize in the TBP-I molecule are also present in the cell surface receptors for TNF.

The effect of the antibodies on binding of TNF to HeLa cells was performed as follows: HeLa cells were seeded into 15 mm tissue culture plates at a density of 2.5 x $10^5$ cells/well. After 24 h incubation at 37°C in 5% CO$_2$ atmosphere, the cells were transferred to ice, the growth medium was removed and the antibodies, diluted in PBS containing 0.5% BSA and 0.1% sodium azide, were applied to the cells for 2 h. The cells were then rinsed and tested for binding of TNF.

### EXAMPLE 9: TNF-like effect of monoclonal antibodies to TBP-I

To obtain additional information on the molecular requirements needed to trigger TNF-like biological activities with antibodies to TBP-I, we examined whether mAbs against TBP-I mediated TNF-like cytotoxicity. Two experimental approaches were taken: in the first, the antibodies were tested for cytotoxicity on SV80 cells without any further treatment (Fig. 11, -anti Ig) and in the second the cytotoxicity of the mAbs was tested after crosslinking them with anti-mouse immunoglobulin antibodies (Fig. 11, + anti Ig). As noted above, all mAbs tested appeared to bind to the class I TNF receptors, yet only two of the 17 mAbs had mild cytotoxic activity on SV80 cells. This difference in biological activity among the mAbs could not be correlated with their isotype nor with their binding capacity in the inverted RIA (Fig. 11). It did appear, however, to relate to the site of binding of the antibodies on the receptor molecule. In cross-competition analysis the two cytotoxic antibodies were found to bind to the same epitope region in TBP-I. None of the other antibodies bound to it (A in Fig. 11). Retesting the effect of the antibodies after cross linking them with anti-immunoglobulin antibodies, we found them all to be highly cytotoxic, to an extent which appeared roughly proportional to the effectiveness at which they bound TBP-I (compare in Fig. 11: cytotoxicity + anti Ig to the binding to TBP-I in inverted RIA).

Testing whether different mAbs to TBP-I can supplement each other in mediating TNF-like cytotoxicity we found that mixtures of two mAbs mapping to different epitope regions on TBP-I were highly cytotoxic to SV80 cells. For example, mixtures of the mAb 18 (epitope region B), which is not cytotoxic by itself, with the non-cytotoxic mAb 20 or 34 (both epitope region C) exerted strong cytotoxicity (app. $3 \times 10^5$ TNF eq. U/mg Ig; see Fig. 11 for definition of unitage). Mixtures of two mAbs mapping to the same epitope region were not more cytotoxic than each mAb alone.

### EXAMPLE 10: The Use of anti-TBP-I antibodies for affinity chromatography

Antibodies to TBP-I can be utilized for the purification of TBP-I by affinity chromatography, as illustrated below with monoclonal antibodies against TBP-I produced as described in Example 6.

The monoclonal antibodies for affinity chromatography were selected by testing their binding capacity for the radiolabeled antigen in a solid phase radio immunoassay. Ascites from all hybridomas was purified by amonium sulfate precipitation at 50% saturation followed by extensive dialysis against PBS. PVC 96 well plates were coated with the purified McAbs as described in Example 9. After blocking the plates with PBS containing 0.5% BSA, 0.05% Tween 20 (Sigma) and 0.02% $NaN_3$, the wells were incubated with 50,000 cpm $^{125}$I-TNF for 2 h et 37°C, then washed and the radioactivity which had bound to each well was quantitated in the gamma-counter. The antibodies with the highest binding capacity were examined for their performance in immuno affinity chromatography.

Polyacryl hydrazide agarose was used as resin to immobilize the antibodies. The semipurified immunoglobulins were concentrated and coupled to the resin as specified by Wilchek and Miron, Methods in Ezymology 34:72-76, 1979. Three monoclonal antibodies against TBP-I, clones 16, 20, and 34 were tested in these experiments.

Antibody columns of 1 ml bed were constructed. Before use, all columns were subjected to 10 washes with the elution buffer, each wash followed by neutralization with PBS. Then the columns were loaded with 120 ml of concentrated urinary proteins in PBS with 0.02% $NaN_3$. The flow rate of the columns was adjusted to 0.2 to 0.3 ml per minute. After loading, the columns were washed with 50 ml PBS and then eluted with a solution containing 50 mM citric acid, pH 2.5, 100 mM NaCl and 0.02% $NaN_3$. Fractions of 1ml were collected. Samples of the applied urinary proteins, the last portion of the wash (1ml) and of each elution fraction (8 fractions of 1ml per column) were taken and tasted for protein concentration (Table V) and activity in the bioassay for TBP-I (Table VI). According to the protein measurements before and after coupling of the antibodies to hydrazide agarose, the following amounts of immunoglobulin had been bound to the columns:

| Clone 16: | 8.4 mg/ml agarose |
|---|---|
| Clone 20: | 7.2 mg/ml agarose |
| Clone 34: | 9.4 mg/ml agarose |

All protein measurements were done according to a micro-flurescamin method in comparison to a standard solution containing 100 $\mu$g BSA/ml (Stein, S. and Moschera, J., Methods Enzymol. 79:7-16, 1981).

The results of bioactivity and protein concentrations of applied, last wash and elution frictions are summarized in tables V and VI.

Table V

| Protein Concentrations | | | |
|---|---|---|---|
| | Clone 16 [$\mu$g/ml] | Clone 20 [$\mu$g/ml] | Clone 34 [$\mu$g/ml] |
| Applied (120 ml) | 35800 | 35800 | 35800 |
| Unbound (120 ml) | 35700 | 35700 | 35700 |
| Last wash (1 ml) | 42 | 38 | 12 |
| Elution 1 (1 ml) | 47 | 38 | 45 |
| Elution 2 (1 ml) | 550 | 784 | 714 |
| Elution 3 (1 ml) | 151 | 150 | 194 |
| Elution 4 (1 ml) | 100 | 51 | 122 |
| Elution 5 (1 ml) | 32 | 24 | 35 |
| The protein concentrations were determined by a micro-flurescamin method in comparison to a BSA standard (100 $\mu$g/ml) | | | |

Table VI

| Purification of TBP-I with monoclonal antibodies bound to hydrazide agarose | | | |
|---|---|---|---|
| | Clone 16 Bioassay [U/ml] | Clone 20 Bioassay [U/ml] | Clone 34 Bioassay [U/ml] |
| Applied | 210 | 210 | 210 |
| Unbound | 100 | 60 | 80 |
| Last wash | *<detect | <detect | <detect |
| Elution 1 | <detect | 850 | 1200 |
| Elution 2 | 4800 | 10100 | 19500 |
| Elution 3 | 3500 | <detect | <detect |
| Elution 4 | 2500 | <detect | <detect |
| Elution 5 | 1000 | <detect | <detect |

* Applied, unbound and last wash were assayed at a dilution of 1:20 and higher and the elution fractions at 1:200 and higher. Samples which gave no discernable signal at this dilution appear as "below detection" (<detect) in the table.

## EXAMPLE 11: Determination of TBP-I using anti-TBP-I antibodies

The levels of TBP-I in the sera of healthy individuals, patients with cancer or systemic lupus erthematosus (SLE) and of pregnant women at term were determined by an ELISA method employing a monoclonal antibody to TBP-I coating the plates. 50 $\mu$l of each sample was added and after a 2.5 h incubation at 37°C the wells were washed with a solution of PBS, Tween 0.05% and sodium azide 0.02%, after which a rabbit anti-TBP-I polyclonal antibody was added for 2.5 h at 37°C. Then the wells were washed again (no azide) and goat anti-rabbit horseradish peroxidase-coupled antibody was added for 2 h. Following this incubation, and washing, an ABTS buffer was added and optical density (O.D.) read 30 min. later at 600nm.

The normal levels of TBP-I in human serum of healthy individuals as determined by the ELISA method are 0.3±0.13 ng/ml.

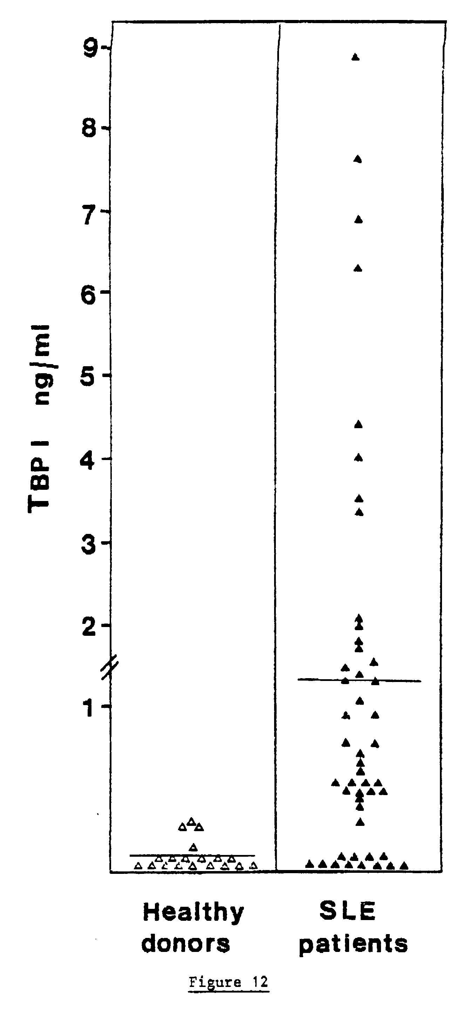

In the sera of 46 patients with Systemic Lupus Erythematosus (SLE), the TBP-I levels were 1.5±1.2 ng/ml, a value highly significant compared to the normal levels ($p < 0.001$). As shown in Fig. 12, 36 out of the 46 patients with SLE had a TBP-I level higher than the mean± 2SD of normal values. We found a highly significant correlation between the TBP-I levels and the disease activity index developed by Symmonds, D.P.M. et al, Quarterly J. of Med. (1988), Vol. 69, pp. 927-937: $r = 0.62$, $p < 0.01$. A similar correlation was found between TBP-I and the classical marker of SLE activity, the anti-DNA antibodies ($r = 0.50$, $p < 0.001$).

These results indicate that TBP-I may be useful as a sensitive marker of disease activity and a predictor of exacerbations in SLE patients, and thus may be useful in monitoring immune activation related to disease activity in these patients as well as in patients with other autoimmune diseases.

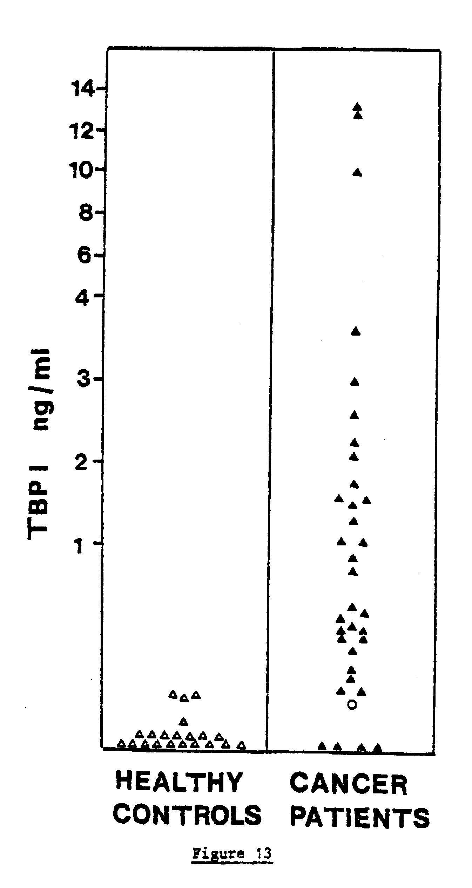

By the above ELISA method, the TBP-I levels in sera of patients with different types of cancer, were examined. In 20 out of 34 patients (58.8%) with different types of cancer, the TBP-I levels were above the normal mean ± 2SD. The difference between the TBP-I of cancer patients (1.5±1.5 ng/ml) and healthy controls (0.3±0.13 ng/ml) was highly significant statistically (p<0.001) - Figure 13.

These results indicate that TSP-I may prove a useful and universal marker of different types of cancer and may be applied in early detection of this condition. After cancer resection, normalization of TBP-I levels may be a marker of cure of the disease. An increase in TBP-I, after initial normalization, may be an early and sensitive universal marker of disease relapse.

**Claims**
**Claims for the following Contracting States : AT, BE, CH, DE, DK, FR, GB, IR, IT, LI, LU, MC, NL, PT, SE**

1. An antibody to the human tumor necrosis factor binding protein TBP-I which specifically recognizes said protein and elicits the same effect in cells as tumor necrosis factor (TNF).

2. The antibody according to claim 1 which is further characterized in that it blocks the binding of TNF to HeLa and MCF7 cells, but does not block the binding of TNF U937 cells.

3. The antibody according to claim 1 or 2 which is a polyclonal antibody.

4. The antibody according to claim 1 or 2 which is a monoclonal antibody.

5. The monoclonal antibody according to claim 4 produced from a hybridoma formed by fusion of myeloma cells with spleen cells and/or lymphocytes of mice previously immunized with TBP-I.

6. The monoclonal antibody according to claim 5 produced from hybridoma TBP-I 18-1 (CNCM I-926).

7. The monoclonal antibody according to claim 5 produced from hybridoma TBP-I 34-6 (CNCM I-927).

8. Hybridoma cell line having the deposit accession number TBP-I 18-1 (CNCM I-926) or TBP-I 34-6 (CNCM I-927).

9. An F(ab) fragment of an antibody according to any one of claims 1 to 7.

10. A pharmaceutical composition comprising as active ingredient an antibody according to any one of claims 1 to 7 or an F(ab) fragment according to claim 9 or salts, functional derivatives or active fractions of the antibody or of the fragment thereof together with a pharmaceutically acceptable carrier.

11. The pharmaceutical composition according to claim 10 containing F(ab) fragments according to claim 9 or salts, functional derivatives or active fractions of the antibody or the fragment thereof, for blocking the binding of TNF to, or inhibiting its effect on cells.

12. The pharmaceutical composition according to claim 10 containing F(ab) fragments according to claim 9 or salts, functional derivatives or active fractions thereof for the treatment of conditions wherein effects of TNF, either endogenously formed or exogenously administered, are to be antagonized.

13. The pharmaceutical composition according to claim 10 for mimicking beneficial effects of TNF on cells.

14. The pharmaceutical composition according to claim 10 containing the antibody according to any one of claims 1 to 7 or salts, functional derivatives or active fractions thereof for mimicking the cytotoxic effect of TNF.

15. An in vitro immunoassay for the TNF binding protein TBP-I in body fluids characterized by measuring its interaction with an antibody to any one of claims 1 to 7.

**16.** A method for the purification of human TNF binding protein TBP-I utilizing an antibody according to any one of claims 1 to 7 or an F(ab) fragment thereof according to claim 9, said method comprising the following steps:

(a) coupling said antibody or fragment thereof to a suitable resin to construct an immunoaffinity column;

(b) loading a solution containing said protein on said immunoaffinity column;

(c) washing away the non-bound proteins with a suitable washing buffer;

(d) eluting the bound TNF binding protein TBP-I with a suitable eluent; and

(e) collecting the enriched fraction of said TBP-I.

**Claims for the following Contracting State : ES**

**1.** A method for the preparation of an antibody to the human tumor necrosis factor binding protein TBP-I which specifically recognizes said protein and elicits the same effect in cells as tumor necrosis factor (TNF), comprising immunization techniques, hybridoma techniques or recombinant DNA techniques.

**2.** The method according to claim 1 wherein said antibody is further characterized in that it blocks the binding of TNF to HeLa and MCF7 cells, but does not block the binding of TNF U937 cells.

**3.** The method according to claim 1 or 2 wherein said antibody is a polyclonal antibody.

**4.** The method according to claim 1 or 2 wherein said antibody is a monoclonal antibody.

**5.** The method according to claim 4 wherein said monoclonal antibody is produced from a hybridoma formed by fusion of myeloma cells with spleen cells and/or lymphocytes of mice previously immunized with TBP-I.

**6.** The method according to claim 5 wherein said monoclonal antibody is produced from hybridoma TBP-I 18-1 (CNCM I-926).

**7.** The method according to claim 5 wherein said monoclonal antibody is produced from hybridoma TBP-I 34-6 (CNCM I-927).

**8.** Hybridoma cell line having the deposit accession number TBP-I 18-1 (CNCM I-926) or TBP-I 34-6 (CNCM I-927).

**9.** A method for the preparation of an F(ab) fragment of an antibody produced according to any one of claims 1 to 7 comprising subjecting the antibody to enzymatic digestion.

**10.** A method for the preparation of a pharmaceutical composition comprising combining as active ingredient an antibody according to any one of claims 1 to 7 or an F(ab) fragment according to claim 9 or salts, functional derivatives or active fractions of the antibody or of the fragment thereof together with a pharmaceutically acceptable carrier.

**11.** The method for the preparation of a pharmaceutical composition according to claim 10 containing F-(ab) fragments according to claim 9 or salts, functional derivatives or active fractions of the antibody or the fragment thereof, wherein said pharmaceutical composition is for blocking the binding of TNF to, or inhibiting its effect on cells.

**12.** The method for the preparation of a pharmaceutical composition according to claim 10 containing F-(ab) fragments according to claim 9 or salts, functional derivatives or active fractions thereof wherein said pharmaceutical composition is for the treatment of conditions wherein effects of TNF, either endogenously formed or exogenously administered, are to be antagonized.

**13.** The method for the preparation of a pharmaceutical composition according to claim 10, wherein said pharmaceutical composition is for mimicking beneficial effects of TNF on cells.

14. The method for the preparation of a pharmaceutical composition according to claim 10 containing the antibody according to any one of claims 1 to 7 or salts, functional derivatives or active fractions thereof wherein said pharmaceutical composition is for mimicking the cytotoxic effect of TNF.

15. An in vitro immunoassay for the TNF binding protein TBP-I in body fluids characterized by measuring its interaction with an antibody to any one of claims 1 to 7.

16. A method for the purification of human TNF binding protein TBP-I utilizing an antibody according to any one of claims 1 to 7 or an F(ab) fragment thereof according to claim 9, said method comprising the following steps:
    (a) coupling said antibody or fragment thereof to a suitable resin to construct an immunoaffinity column;
    (b) loading a solution containing said protein on said immunoaffinity column;
    (c) washing away the non-bound proteins with a suitable washing buffer;
    (d) eluting the bound TNF binding protein TBP-I with a suitable eluent; and
    (e) collecting the enriched fraction of said TBP-I.

**Claims for the following Contracting State : GR**

1. An antibody to the human tumor necrosis factor binding protein TBP-I which specifically recognizes said protein and elicits the same effect in cells as tumor necrosis factor (TNF).

2. The antibody according to claim 1 which is further characterized in that it blocks the binding of TNF to HeLa and MCF7 cells, but does not block the binding of TNF U937 cells.

3. The antibody according to claim 1 or 2 which is a polyclonal antibody.

4. The antibody according to claim 1 or 2 which is a monoclonal antibody.

5. The monoclonal antibody according to claim 4 produced from a hybridoma formed by fusion of myeloma cells with spleen cells and/or lymphocytes of mice previously immunized with TBP-I.

6. The monoclonal antibody according to claim 5 produced from hybridoma TBP-I 18-1 (CNCM I-926).

7. The monoclonal antibody according to claim 5 produced from hybridoma TBP-I 34-6 (CNCM I-927).

8. Hybridoma cell line having the deposit accession number TBP-I 18-1 (CNCM I-926) or TBP-I 34-6 (CNCM I-927).

9. An F(ab) fragment of an antibody according to any one of claims 1 to 7.

10. A method for the preparation of a pharmaceutical composition comprising combining as active ingredient an antibody according to any one of claims 1 to 7 or an F(ab) fragment according to claim 9 or salts, functional derivatives or active fractions of the antibody or of the fragment thereof together with a pharmaceutically acceptable carrier.

11. The method for the preparation of a pharmaceutical composition according to claim 10 containing F-(ab) fragments according to claim 9 or salts, functional derivatives or active fractions of the antibody or the fragment thereof, wherein said pharmaceutical composition is for blocking the binding of TNF to, or inhibiting its effect on cells.

12. The method for the preparation of a pharmaceutical composition according to claim 10 containing F-(ab) fragments according to claim 9 or salts, functional derivatives or active fractions thereof wherein said pharmaceutical composition is for the treatment of conditions wherein effects of TNF, either endogenously formed or exogenously administered, are to be antagonized.

13. The method for the preparation of a pharmaceutical composition according to claim 10, wherein said pharmaceutical composition is for mimicking beneficial effects of TNF on cells.

**14.** The method for the preparation of a pharmaceutical composition according to claim 10 containing the antibody according to any one of claims 1 to 7 or salts, functional derivatives or active fractions thereof wherein said pharmaceutical composition is for mimicking the cytotoxic effect of TNF.

**15.** An in vitro immunoassay for the TNF binding protein TBP-I in body fluids characterized by measuring its interaction with an antibody to any one of claims 1 to 7.

**16.** A method for the purification of human TNF binding protein TBP-I utilizing an antibody according to any one of claims 1 to 7 or an F(ab) fragment thereof according to claim 9, said method comprising the following steps:
   (a) coupling said antibody or fragment thereof to a suitable resin to construct an immunoaffinity column;
   (b) loading a solution containing said protein on said immunoaffinity column;
   (c) washing away the non-bound proteins with a suitable washing buffer;
   (d) eluting the bound TNF binding protein TBP-I with a suitable eluent; and
   (e) collecting the enriched fraction of said TBP-I.

**Patentansprüche**
**Patentansprüche für folgende Vertragsstaaten : AT, BE, CH, DE, DK, FR, GB, IR, IT, LI, LU, MC, NL, PT, SE**

**1.** Antikörper gegen das menschlichen Tumornekrose-Faktor bindende Protein TBP-I, der dieses Protein spezifisch erkennt und in Zellen die gleiche Wirkung hat wie Tumornekrose-Faktor (TNF).

**2.** Antikörper nach Anspruch 1, dadurch gekennzeichnet, daß er die Bindung von TNF an HeLa- und MCF7-Zellen, jedoch nicht an TNF U937-Zellen, hemmt.

**3.** Antikörper nach Anspruch 1 oder 2, der ein polyclonaler Antikörper ist.

**4.** Antikörper nach Anspruch 1 oder 2, der ein monoclonaler Antikörper ist.

**5.** Monoclonaler Antikörper nach Anspruch 4, der von einem Hybridom erzeugt wird, das durch Fusion von Myelomzellen mit Milzzellen und/oder Lymphocyten von Mäusen hergestellt wird, die zuvor mit TBP-I immunisiert wurden.

**6.** Monoclonaler Antikörper nach Anspruch 5, der von dem Hybridom TBP-I 18-1 (CNCM I-926) erzeugt wird.

**7.** Monoclonaler Antikörper nach Anspruch 5, der von dem Hybridom TBP-I 34-6 (CNCM I-927) erzeugt wird.

**8.** Hybridomzellinie mit der Hinterlegungsnummer TBP-I 18-1 (CNCM I-926) oder TBP-I 34-6 (CNCM I-927).

**9.** F(ab)-Fragment eines Antikörpers nach einem der Ansprüche 1 bis 7.

**10.** Arzneimittel, enthaltend als Wirkstoff einen Antikörper nach einem der Ansprüche 1 bis 7 oder ein F-(ab)-Fragment nach Anspruch 9 oder Salze, funktionelle Derivate oder aktive Fraktionen des Antikörpers oder dessen Fragments zusammen mit einem pharmazeutisch verträglichen Träger.

**11.** Arzneimittel nach Anspruch 10, enthaltend F(ab)-Fragmente nach Anspruch 9 oder Salze, funktionelle Derivate oder aktive Fraktionen des Antikörpers oder dessen Fragments zur Hemmung der Bindung von TNF an Zellen oder zur Hemmung von dessen Wirkung auf Zellen.

**12.** Arzneimittel nach Anspruch 10, enthaltend F(ab)-Fragmente nach Anspruch 9 oder deren Salze, funktionelle Derivate oder aktive Fraktionen zur Behandlung von Krankheitszuständen, bei denen den Wirkungen von entweder endogen gebildetem oder exogen verabreichtem TNF entgegengewirkt werden soll.

**13.** Arzneimittel nach Anspruch 10 zur Nachahmung der vorteilhaften Wirkungen von TNF auf Zellen.

**14.** Arzneimittel nach Anspruch 10, enthaltend den Antikörper nach einem der Ansprüche 1 bis 7 oder dessen Salze, funktionelle Derivate oder aktive Fraktionen zur Nachahmung der cytotoxischen Wirkung von TNF.

**15.** In vitro-Immuntest für das TNF-bindende Protein TBP-I in Körperflüssigkeiten, dadurch gekennzeichnet, daß seine Wechselwirkung mit einem Antikörper nach einem der Ansprüche 1 bis 7 gemessen wird.

**16.** Verfahren zur Reinigung des menschlichen TNF-bindenden Proteins TBP-I unter Verwendung eines Antikörpers nach einem der Ansprüche 1 bis 7 oder eines F(ab)-Fragments davon nach Anspruch 9, wobei das Verfahren die folgenden Schritte umfaßt:
(a) Kopplung des Antikörpers oder des Fragments davon an ein geeignetes Harz zur Herstellung einer Immunaffinitätssäule,
(b) Beladen dieser Immunaffinitätssäule mit einer dieses Protein enthaltenden Lösung,
(c) Entfernen der nicht-gebundenen Proteine mit einem geeigneten Waschpuffer,
(d) Eluieren des gebundenen TNF-bindenden Proteins TBP-I mit einem geeigneten Elutionsmittel, und
(e) Gewinnen der mit TBP-I angereicherten Fraktion.

**Patentansprüche für folgenden Vertragsstaat : ES**

**1.** Verfahren zur Herstellung eines Antikörpers gegen das menschlichen Tumornekrose-Faktor bindende Protein TBP-I, der dieses Protein spezifisch erkennt und in Zellen die gleiche Wirkung hat wie Tumornekrose-Faktor (TNF), umfassend Immunisierungstechniken, Hybridomtechniken oder rekombinante DNA-Techniken.

**2.** Verfahren nach Anspruch 1, wobei der Antikörper weiter dadurch gekennzeichnet ist, daß er die Bindung von TNF an HeLa- und MCF7-Zellen, jedoch nicht an TNF U937-Zellen hemmt.

**3.** Verfahren nach Anspruch 1 oder 2, wobei der Antikörper ein polyclonaler Antikörper ist.

**4.** Verfahren nach Anspruch 1 oder 2, wobei der Antikörper ein monoclonaler Antikörper ist.

**5.** Verfahren nach Anspruch 4, wobei der monoclonale Antikörper von einem Hybridom erzeugt wird, das durch Fusion von Myelomzellen mit Milzzellen und/oder Lymphocyten von Mäusen hergestellt wird, die zuvor mit TBP-I immunisiert wurden.

**6.** Verfahren nach Anspruch 5, wobei der monoclonale Antikörper von dem Hybridom TBP-I 18-1 (CNCM I-926) erzeugt wird.

**7.** Verfahren nach Anspruch 5, wobei der monoclonale Antikörper von dem Hybridom TBP-I 34-6 (CNCM I-927) erzeugt wird.

**8.** Hybridomzellinie mit der Hinterlegungsnummer TBP-I 18-1 (CNCM I-926) oder TBP-I 34-6 (CNCM I-927).

**9.** Verfahren zur Herstellung eines F(ab)-Fragments eines Antikörpers, der nach einem der Ansprüche 1 bis 7 hergestellt wurde, umfassend den enzymatischen Abbau des Antikörpers.

**10.** Verfahren zur Herstellung eines Arzneimittels, umfassend die Kombination eines Antikörpers nach einem der Ansprüche 1 bis 7 oder eines F(ab)-Fragments nach Anspruch 9 oder von Salzen, funktionellen Derivaten oder aktiven Fraktionen des Antikörpers oder dessen Fragments als Wirkstoff mit einem pharmazeutisch verträglichen Träger.

**11.** Verfahren zur Herstellung eines Arzneimittels nach Anspruch 10, enthaltend F(ab)-Fragmente nach Anspruch 9 oder Salze, funktionelle Derivate oder aktive Fraktionen des Antikörpers oder dessen Fragments, wobei das Arzneimittel zur Hemmung der Bindung von TNF an Zellen oder zur Hemmung von dessen Wirkung auf Zellen dient.

**12.** Verfahren zur Herstellung eines Arzneimittels nach Anspruch 10, enthaltend F(ab)-Fragmente nach Anspruch 9 oder deren Salze, funktionelle Derivate oder aktive Fraktionen, wobei das Arzneimittel zur Behandlung von Krankheitszuständen dient, bei denen den Wirkungen von entweder endogen gebildetem oder exogen verabreichtem TNF entgegengewirkt werden soll.

**13.** Verfahren zur Herstellung eines Arzneimittels nach Anspruch 10, wobei das Arzneimittel zur Nachahmung der vorteilhaften Wirkungen von TNF auf Zellen dient.

**14.** Verfahren zur Herstellung eines Arzneimittels nach Anspruch 10, enthaltend den Antikörper nach einem der Ansprüche 1 bis 7 oder dessen Salze, funktionelle Derivate oder aktive Fraktionen, wobei das Arzneimittel zur Nachahmung der cytotoxischen Wirkung von TNF dient.

**15.** In vitro-Immuntest für das TNF-bindende Protein TBP-I in Körperflüssigkeiten, dadurch gekennzeichnet, daß seine Wechselwirkung mit einem Antikörper nach einem der Ansprüche 1 bis 7 gemessen wird.

**16.** Verfahren zur Reinigung des menschlichen TNF-bindenden Proteins TBP-I unter Verwendung eines Antikörpers nach einem der Ansprüche 1 bis 7 oder eines F(ab)-Fragments davon nach Anspruch 9, wobei das Verfahren die folgenden Schritte umfaßt:
(a) Kopplung des Antikörpers oder des Fragments davon an ein geeignetes Harz zur Herstellung einer Immunaffinitätssäule,
(b) Beladen dieser Immunaffinitätssäule mit einer dieses Protein enthaltenden Lösung,
(c) Entfernen der nicht-gebundenen Proteine mit einem geeigneten Waschpuffer,
(d) Eluieren des gebundenen TNF-bindenden Proteine TBP-I mit einem geeigneten Elutionsmittel, und
(e) Gewinnen der mit TBP-I angereicherten Fraktion.

**Patentansprüche für folgenden Vertragsstaat : GR**

**1.** Antikörper gegen das menschlichen Tumornekrose-Faktor bindende Protein TBP-I, der dieses Protein spezifisch erkennt und in Zellen die gleiche Wirkung hat wie Tumornekrose-Faktor (TNF).

**2.** Antikörper nach Anspruch 1, dadurch gekennzeichnet, daß er die Bindung von TNF an HeLa- und MCF7-Zellen, jedoch nicht an TNF U937-Zellen hemmt.

**3.** Antikörper nach Anspruch 1 oder 2, der ein polyclonaler Antikörper ist.

**4.** Antikörper nach Anspruch 1 oder 2, der ein monoclonaler Antikörper ist.

**5.** Monoclonaler Antikörper nach Anspruch 4, der von einem Hybridom erzeugt wird, das durch Fusion von Myelomzellen mit Milzzellen und/oder Lymphocyten von Mäusen hergestellt wird, die zuvor mit TBP-I immunisiert wurden.

**6.** Monoclonaler Antikörper nach Anspruch 5, der von dem Hybridom TBP-I 18-1 (CNCM I-926) erzeugt wird.

**7.** Monoclonaler Antikörper nach Anspruch 5, der von dem Hybridom TBP-I 34-6 (CNCM I-927) erzeugt wird.

**8.** Hybridomzellinie mit der Hinterlegungsnummer TBP-I 18-1 (CNCM I-926) oder TBP-I 34-6 (CNCM I-927).

**9.** F(ab)-Fragment eines Antikörpers nach einem der Ansprüche 1 bis 7.

**10.** Verfahren zur Herstellung eines Arzneimittels, umfassend die Kombination eines Antikörpers nach einem der Ansprüche 1 bis 7 oder eines F(ab)-Fragments nach Anspruch 9 oder von Salzen, funktionellen Derivaten oder aktiven Fraktionen des Antikörpers oder dessen Fragments als Wirkstoff

EP 0 412 486 B1

mit einem pharmazeutisch verträglichen Träger.

**11.** Verfahren zur Herstellung eines Arzneimittels nach Anspruch 10, enthaltend F(ab)-Fragmente nach Anspruch 9 oder Salze, funktionelle Derivate oder aktive Fraktionen des Antikörpers oder dessen Fragments, wobei das Arzneimittel zur Hemmung der Bindung von TNF an Zellen oder zur Hemmung von dessen Wirkung auf Zellen dient.

**12.** Verfahren zur Herstellung eines Arzneimittels nach Anspruch 10, enthaltend F(ab)-Fragmente nach Anspruch 9 oder deren Salze, funktionelle Derivate oder aktive Fraktionen, wobei das Arzneimittel zur Behandlung von Krankheitszuständen dient, bei denen den Wirkungen von entweder endogen gebildetem oder exogen verabreichtem TNF entgegengewirkt werden soll.

**13.** Verfahren zur Herstellung eines Arzneimittels nach Anspruch 10, wobei das Arzneimittel zur Nachahmung der vorteilhaften Wirkungen von TNF auf Zellen dient.

**14.** Verfahren zur Herstellung eines Arzneimittels nach Anspruch 10, enthaltend den Antikörper nach einem der Ansprüche 1 bis 7 oder dessen Salze, funktionelle Derivate oder aktive Fraktionen, wobei das Arzneimittel zur Nachahmung der cytotoxischen Wirkung von TNF dient.

**15.** In vitro-Immuntest für das TNF-bindende Protein TBP-I in Körperflüssigkeiten, dadurch gekennzeichnet, daß seine Wechselwirkung mit einem Antikörper nach einem der Ansprüche 1 bis 7 gemessen wird.

**16.** Verfahren zur Reinigung des menschlichen TNF-bindenden Proteine TBP-I unter Verwendung eines Antikörpers nach einem der Ansprüche 1 bis 7 oder eines F(ab)-Fragments davon nach Anspruch 9, wobei das Verfahren die folgenden Schritte umfaßt:
(a) Kopplung des Antikörpers oder des Fragments davon an ein geeignetes Harz zur Herstellung einer Immunaffinitätssäule,
(b) Beladen dieser Immunaffinitätssäule mit einer dieses Protein enthaltenden Lösung,
(c) Entfernen der nicht-gebundenen Proteine mit einem geeigneter Waschpuffer,
(d) Eluieren des gebundenen TNF-bindenden Proteins TBP-I mit einem geeigneten Elutionsmittel, und
(e) Gewinnen der mit TBP-I angereicherten Fraktion.

**Revendications**

**Revendications pour les Etats contractants suivants : AT, BE, CH, DE, DK, FR, GB, IR, IT LI, LU, MC, NL, PT, SE**

**1.** Anticorps contre la protéine humaine de liaison TBP-I du facteur de nécrose tumorale, qui reconnaît spécifiquement ladite protéine et provoque le même effet dans les cellules que le facteur de nécrose tumorale (TNF).

**2.** Anticorps selon la revendication 1, qui est caractérisé en outre en ce qu'il empêche la liaison du TNF aux cellules HeLa et MCF7, mais n'empêche pas la liaison des cellules TNF U937.

**3.** Anticorps selon la revendication 1 ou 2, qui est un anticorps polyclonal.

**4.** Anticorps selon la revendication 1 ou 2, qui est un anticorps monoclonal.

**5.** Anticorps monoclonal selon la revendication 4, produit à partir d'un hybridome formé par fusion de cellules de myélome avec des cellules de rate et/ou des lymphocytes de souris préalablement immunisés avec de la TBP-I.

**6.** Anticorps monoclonal selon la revendication 5, produit à partir de l'hybridome TBP-I 18-1 (CNCM I-926).

**7.** Anticorps monoclonal selon la revendication 5, produit à partir de l'hybridome TBP-I 34-6 (CNCM I-927).

24

8. Lignée de cellule d'hybridome ayant le numéro d'accès TBP-I 18-1 (CNCM I-926) ou TPI-I 34-6 (CNCM I-927).

9. Fragment F(ab) d'un anticorps selon l'une quelconque des revendications 1 à 7.

10. Composition pharmaceutique comprenant comme ingrédient actif un anticorps selon l'une quelconque des revendications 1 à 7, ou un fragment F(ab) selon la revendication 9 ou des sels, des dérivés fonctionnels ou des fractions actives de l'anticorps ou de son fragment en même temps qu'un porteur pharmaceutiquement acceptable.

11. Composition pharmaceutique selon la revendication 10, contenant des fragments F(ab) selon la revendication 9 ou des sels, des dérivés fonctionnels ou des fractions actives de l'anticorps ou de son fragment, pour empêcher la liaison du TNF aux cellules ou inhiber son effet sur celles-ci.

12. Composition pharmaceutique selon la revendication 10, contenant des fragments F(ab) selon la revendication 9 ou des sels, des dérivés fonctionnels ou des fractions actives de ceux-ci, pour le traitement d'états où les effets du TNF, soit formé de façon endogène, soit administré de façon exogène, doivent être antagonisés.

13. Composition pharmaceutique selon la revendication 10, pour simuler les effets bénéfiques du TNF sur les cellules.

14. Composition pharmaceutique selon la revendication 10, contenant un anticorps selon l'une quelconque des revendications 1 à 7 ou des sels, des dérivés fonctionnels ou des fractions actives de celui-ci, pour simuler l'effet cytotoxique du TNF.

15. Immunoessai in vitro pour la protéine de liaison TBP-I du TNF dans les fluides corporels, caractérisé par la mesure de son interaction avec un anticorps selon l'une quelconque des revendications 1 à 7.

16. Procédé pour la purification de la protéine humaine de liaison TBP-I du TNF utilisant un anticorps selon l'une quelconque des revendications 1 à 7 ou un fragment F(ab) de celui-ci selon la revendication 9, ledit procédé comprenant les étapes suivantes consistant à :
   (a) coupler ledit anticorps ou un fragment de celui-ci à une résine appropriée pour former une colonne d'immunoaffinité ;
   (b) charger une solution contenant ladite protéine sur ladite colonne d'immunoaffinité ;
   (c) éliminer par lavage les protéines non liées avec un tampon de lavage approprié ;
   (d) éluer la protéine de liaison TBP-I du TNF liée avec un éluant approprié ; et
   (e) collecter la fraction enrichie de ladite TBP-I.

**Revendications pour l'Etat contractant suivant : ES**

1. Procédé pour la préparation d'un anticorps contre la protéine humaine de liaison TBP-I du facteur de nécrose tumorale, qui reconnaît spécifiquement ladite protéine et provoque le même effet dans les cellules que le facteur de nécrose tumorale (TNF), comprenant des techniques d'immunisation, des techniques l'hybridome ou des techniques de l'ADN recombinant.

2. Procédé selon la revendication 1, dans laquelle ledit anticorps est caractérisé en outre en ce qu'il empêche la liaison du TNF aux cellules HeLa et MCF7, mais n'empêche pas la liaison des cellules TNF U937.

3. Procédé selon la revendication 1 ou 2, dans laquelle ledit anticorps est un anticorps polyclonal.

4. Procédé selon la revendication 1 ou 2, dans laquelle ledit anticorps est un anticorps monoclonal.

5. Procédé selon la revendication 4, dans laquelle ledit anticorps monoclonal est produit à partir d'un hybridome formé par fusion de cellules de myélome avec des cellules de rate et/ou des lymphocytes de souris préalablement immunisés avec de la TBP-I.

**6.** Procédé selon la revendication 5, dans laquelle ledit anticorps monoclonal est produit à partir de l'hybridome TBP-I 18-1 (CNCM I-926).

**7.** Procédé selon la revendication 5, dans laquelle ledit anticorps monoclonal est produit à partir de l'hybridome TBP-I 34-6 (CNCM I-927).

**8.** Lignée de cellule d'hybridome ayant le numéro d'accès TBP-I 18-1 (CNCM I-926) ou TBP-I 34-6 (CNCM I-927).

**9.** Procédé pour la préparation d'un fragment F(ab) d'un anticorps selon l'une quelconque des revendications 1 à 7, comprenant la submission de l'anticorps à une digestion enzymatique.

**10.** Procédé pour la préparation d'une composition pharmaceutique comprenant la combination, comme ingrédient actif, d'un anticorps selon l'une quelconque des revendications 1 à 7, ou d'un fragment F(ab) selon la revendication 9 ou des sels, des dérivés fonctionels ou des fractions actives de l'anticorps ou de son fragment en même temps qu'un porteur pharmaceutiquement acceptable.

**11.** Procédé pour la préparation d'une composition pharmaceutique selon la revendication 10, contenant des fragments F(ab) selon la revendication 9 ou des sels, des dérivés fonctionnels ou des fractions actives de l'anticorps ou de son fragment,
dans laquelle ladite composition pharmaceutique empêche la liaison du TNF aux cellules ou inhiber son effet sur celles-ci.

**12.** Procédé pour la préparation d'une composition pharmaceutique selon la revendication 10, contenant des fragments F(ab) selon la revendication 9 our des sels, des dérivés fonctionnels ou des fractions actives de ceux-ci, dans laquelle ladite composition pharmaceutique traite des états où les effects du TNF, soit formé de façon endogène, soit administré de façon exogène, doivent être antagonisés.

**13.** Procédé pour la préparation d'une composition pharmaceutique selon la revendication 10 dans laquelle ladite composition pharmaceutique simule les effects bénéfiques du TNF sur les cellules.

**14.** Procédé pour la préparation d'une composition pharmaceutique selon la revendication 10, contenant un anticorps selon l'une quelconque des revendications 1 à 7 ou des sels, des dérivés fonctionnels ou des fractions actives de celui-ci, dans laquelle ladite composition pharmaceutique simule l'effects cytotoxique du TNF.

**15.** Immunoessai in vitro pour la protéine de liaison TBP-I du TNF dans les fluides corporels, caractérisé par la mesure de son interaction avec un anticorps selon l'une quelconque des revendications 1 à 7.

**16.** Procédé pour la purification de la protéine humaine de liaison TBP-I du TNF utilisant un anticorps selon l'une quelconque des revendications 1 à 7 ou un fragment F(ab) de celui-ci selon la revendication 9, ledit procédé comprenant les étapes suivantes consistant à :
 (a) coupler ledit anticorps ou un fragment de celui-ci à une résine appropriée pour former une colonne d'immunoaffinité ;
 (b) charger une solution contenant ladite protéine sur ladite colonne d'immunoaffinité ;
 (c) éliminer par lavage les protéines non liées avec un tampon de lavage approprié ;
 (d) éluer la protéine de liaison TBP-I du TNF liée avec un éluant approprié ; et
 (e) collecter la fraction enrichie de ladite TBP-I.

**Revendications pour l'Etat contractant suivant : GR**

**1.** Anticorps contre la protéine humaine de liaison TBP-I du facteur de nécrose tumorale, qui reconnaît spécifiquement ladite protéine et provoque le même effet dans les cellules que le facteur de nécrose tumorale (TNF).

**2.** Anticorps selon la revendication 1, qui est caractérisé en outre en ce qu'il empêche la liaison du TNF aux cellules HeLa et MCF7, mais n'empêche pas la liaison des cellules TNF U937.

3. Anticorps selon la revendication 1 ou 2, qui est un anticorps polyclonal.

4. Anticorps selon la revendication 1 ou 2, qui est un anticorps monoclonal.

5. Anticorps monoclonal selon la revendication 4, produit à partir d'un hybridome formé par fusion de cellules de myélome avec des cellules de rate et/ou des lymphocytes de souris préalablement immunisés avec de la TBP-I.

6. Anticorps monoclonal selon la revendication 5, produit à partir de l'hybridome TBP-I 18-1 (CNCM I-926).

7. Anticorps monoclonal selon la revendication 5, produit à partir de l'hybridome TBP-I 34-6 (CNCM I-927).

8. Lignée de cellule d'hybridome ayant le numéro d'accès TBP-I 18-1 (CNCM I-926) ou TBP-I 34-6 (CNCM I-927).

9. Fragment F(ab) d'un anticorps selon l'une quelconque des revendications 1 à 7.

10. Procédé pour la préparation d'une composition pharmaceutique comprenant la combinaison, comme ingrédient actif, d'un anticorps selon l'une quelconque des revendications 1 à 7, ou d'un fragment F(ab) selon la revendication 9 ou des sels, des dérivés fonctionels ou des fractions actives de l'anticorps ou de son fragment en même temps qu'un porteur pharmaceutiquement acceptable.

11. Procédé pour la préparation d'une composition pharmaceutique selon la revendication 10, contenant des fragments F(ab) selon la revendication 9 ou des sels des dérivés fonctionnels ou des fractions actives de l'anticorps ou de son fragment,
dans laquelle ladite composition pharmaceutique empêche la liaison du TNF aux cellules ou inhiber son effet sur celles-ci.

12. Procédé pour la préparation d'une composition pharmaceutique selon la revendication 10, contenant des fragments F(ab) selon la revendication 9 our des sels, des dérivés fonctionnels ou des fractions actives de ceux-ci, dans laquelle ladite composition pharmaceutique traite des états où les effects du TNF, soit formé de façon endogène, soit administré de façon exogène, doivent être antagonisés.

13. Procédé pour la préparation d'une composition pharmaceutique selon la revendication 10 dans laquelle ladite composition pharmaceutique simule les effects bénéfiques du TNF sur les cellules.

14. Procédé pour la préparation d'une composition pharmaceutique selon la revendication 10, contenant un anticorps selon l'une quelconque des revendications 1 à 7 ou des sels, des dérivés fonctionnels ou des fractions actives de celui-ci, dans laquelle ladite composition pharmaceutique simule l'effects cytotoxique du TNF.

15. Immunoessai in vitro pour la protéine de liaison TBP-I du TNF dans les fluides corporels, caractérisé par la mesure de son interaction avec un anticorps selon l'une quelconque des revendications 1 à 7.

16. Procédé pour la purification de la protéine humaine de liaison TBP-I du TNF utilisant un anticorps selon l'une quelconque des revendications 1 à 7 ou un fragment F(ab) de celui-ci selon la revendication 9, ledit procédé comprenant les étapes suivantes consistant à :
(a) coupler ledit anticorps ou un fragment de celui-ci à une résine appropriée pour former une colonne d'immunoaffinité ;
(b) charger une solution contenant ladite protéine sur ladite colonne d'immunoaffinité ;
(c) éliminer par lavage les protéines non liées avec un tampon de lavage approprié ;
(d) éluer la protéine de liaison TBP-I du TNF liée avec un éluant approprié ; et
(e) collecter la fraction enrichie de ladite TBP-I.

FIGURE 1

28

FIGURE 2

FIGURE 3

FIGURE 4

FIGURE 5

_FIGURE_6_

33

$^3$H-thymidine incorporation [cpm]

A

serum dilution

B

TNF concentration [pg/ml]

FIGURE 7

FIGURE 8

FIGURE 9A

FIGURE 9B

FIGURE 9C

F(ab)

aIgG

aTBPI

F(ab)+ aIgG

FIGURE 10

EP 0 412 486 B1

**FIGURE 11**

### Cross competition analysis

Binding of TBP I in inverted RIA (at 200 ng Ig/ml) [cpm bound]

antibody bound to solid phase: 17 23 18 20 34 30 68

competitor antibody (in solution): A (17, 23), B (18), C (20, 34), D (30, 68)

| Monoclonal antibody | Isotype | Binding of TBP I in inverted RIA [cpm bound] | Suppression of TNF binding to HeLa cells (at 1 µg Ig/ml) | TNF-like cytotoxicity anti Ig − [TNF eq. U/mg Ig] | TNF-like cytotoxicity anti Ig + [TNF eq. U/mg Ig] |
|---|---|---|---|---|---|
| 17 | IgG$_{2a}$ | 1300 | 100% | $2.0\times10^{1}$ | $9.0\times10^{4}$ |
| 23 | IgG$_{2b}$ | 800 | 100% | $3.4\times10^{2}$ | $8.3\times10^{4}$ |
| 18 | IgG$_{2b}$ | 12300 | 100% | n.ctx. | $3.3\times10^{5}$ |
| 20 | IgG$_{2a}$ | 17900 | 100% | n.ctx. | $1.5\times10^{5}$ |
| 34 | IgG$_{2a}$ | 15000 | 100% | n.ctx. | $1.6\times10^{5}$ |
| 30 | IgG$_{2a}$ | 8000 | 100% | n.ctx. | $1.5\times10^{5}$ |
| 68 | IgG$_{2a}$ | 6000 | 100% | n.ctx. | $9.0\times10^{4}$ |

**[%binding]**

<25  25 to 50  50 to 75  75 to 100  >125

Figure 12

Figure 13